# EUROPEAN PATENT APPLICATION

(11) **EP 1 847 600 A1**
(43) Date of publication of application: **24.10.2007**
(21) Application number: 06112706.4
(22) Date of filing: 18.04.2006
(51) Int. Cl.: C12N 9/76, A61K 38/48, C07K 14/525

(54) **Specific protease for inactivation of human tumour necrosis factor-alpha**

(71) Applicant: Direvo Biotech AG, 50829 Köln (DE)
(72) Inventor: Kettling, Ulrich, 50829 Köln (DE); Koltermann, André, 50829 Köln (DE); Haupts, Ulrich, 50829 Köln (DE); Rarbach, Markus, 50829 Köln (DE); Tebbe, Jan, 50829 Köln (DE); Votsmeier, Christian, 50829 Köln (DE); Scheidig, Andreas, 50829 Köln (DE); Coco, Wayne M., 50829 Köln (DE); Da Silva, Antonio, 50829 Köln (DE); Hesse, Oliver, 50829 Köln (DE); Scholz, Peter, 50829 Köln (DE); Gritzan, Uwe, 50829 Köln (DE); Zeidler, Georg, 50829 Köln (DE); Kalmbach, Rolf, 50829 Köln (DE)
(74) Representative: Helbing, Jörg

(57) **Abstract**

The present invention is directed to specific proteases, fragments and derivatives thereof that specifically inactivate tumour necrosis factor-alpha, methods for their preparation, pharmaceutical and diagnostic compositions comprising such a protease. The proteases of the invention are useful for the treatment of clinical conditions resulting from the detrimental activity of tumour necrosis factor. The invention further relates to nucleotide sequences thereof encoding the protease, as well as vectors and hosts containing such sequences.

SEQUENCE LISTING

<110>  Direvo Biotech AG

<120>  Specific Protease for Inactivation of Human Tumour Necrosis
       Factor-alpha

<130>  060447ep

<160>  34

<170>  PatentIn version 3.3

<210>  1
<211>  224
<212>  PRT
<213>  Homo sapiens

<400>  1

Ile Val Gly Gly Tyr Asn Cys Glu Glu Asn Ser Val Pro Tyr Gln Val
1               5                   10                  15

Ser Leu Asn Ser Gly Tyr His Phe Cys Gly Gly Ser Leu Ile Asn Glu
            20                  25                  30

Gln Trp Val Val Ser Ala Gly His Cys Tyr Lys Ser Arg Ile Gln Val
        35                  40                  45

Arg Leu Gly Glu His Asn Ile Glu Val Leu Glu Gly Asn Glu Gln Phe
    50                  55                  60

Ile Asn Ala Ala Lys Ile Ile Arg His Pro Gln Tyr Asp Arg Lys Thr
65                  70                  75                  80

Leu Asn Asn Asp Ile Met Leu Ile Lys Leu Ser Ser Arg Ala Val Ile
                85                  90                  95

Asn Ala Arg Val Ser Thr Ile Ser Leu Pro Thr Ala Pro Pro Ala Thr
            100                 105                 110

Gly Thr Lys Cys Leu Ile Ser Gly Trp Gly Asn Thr Ala Ser Ser Gly
        115                 120                 125

Ala Asp Tyr Pro Asp Glu Leu Gln Cys Leu Asp Ala Pro Val Leu Ser
    130                 135                 140

Gln Ala Lys Cys Glu Ala Ser Tyr Pro Gly Lys Ile Thr Ser Asn Met
145                 150                 155                 160

Phe Cys Val Gly Phe Leu Glu Gly Gly Lys Asp Ser Cys Gln Gly Asp
                165                 170                 175

Ser Gly Gly Pro Val Val Cys Asn Gly Gln Leu Gln Gly Val Val Ser
            180                 185                 190

Trp Gly Asp Gly Cys Ala Gln Lys Asn Lys Pro Gly Val Tyr Thr Lys
        195                 200                 205

Val Tyr Asn Tyr Val Lys Trp Ile Lys Asn Thr Ile Ala Ala Asn Ser
    210                 215                 220

<210>  2
<211>  238
<212>  PRT
<213>  artificial

<220>
<223>  variant E

<400>  2

Ile Val Gly Gly Tyr Asp Cys Glu Glu Asn Ser Val Pro Tyr His Val
1               5                   10                  15

Ser Leu Asn Ser Gly Ser Asn Ser Thr Thr Ile Cys Gly Gly Ala Leu
            20                  25                  30

Ile Asn Glu Gln Trp Val Val Thr Ala Gly His Cys Tyr Ala Phe Phe
        35                  40                  45

Pro Ala Asp Lys Ser Arg Ile Gln Val Arg Leu Gly Val His Ser Thr
    50                  55                  60

Gly Ala Leu Glu Gly Asn Glu Gln Ile Ile Asn Ala Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Ser Pro Ala Val Ile Asn Ala His Val Ser Thr Ile
            100                 105                 110

Ser Leu Pro Thr Ala Pro Pro Ala Thr Gly Thr Lys Cys Leu Ile Ser
        115                 120                 125

Gly Trp Gly Asn Ser Lys Asp Pro Tyr Lys Pro Ser Ser Arg Ala Asp
    130                 135                 140

Tyr Pro Gly Glu Val Gln Cys Leu Asp Ala Pro Val Leu Ser Gln Thr
145                 150                 155                 160

Lys Cys Glu Ala Ser Tyr Pro Gly Lys Ile Thr Ser Asn Met Phe Cys
                165                 170                 175

Ala Gly Phe Leu Glu Gly Gly Lys Asp Ser Cys Gln Gly Asp Ser Gly
            180                 185                 190

Gly Pro Val Val His Asn Gly Gln Leu Gln Gly Val Val Ser Trp Gly
        195                 200                 205

Glu Gly Cys Ala Gln Lys Asn Lys Pro Gly Val Tyr Thr Asn Val Tyr
    210                 215                 220

Asn Tyr Val Glu Trp Ile Gln Asp Thr Ile Ala Ala Asn Ser
225                 230                 235

<210>  3
<211>  238
<212>  PRT
<213>  artificial

<220>
<223>  variant F

<400>  3

Ile Val Gly Gly Tyr Asp Cys Glu Glu Asn Ser Val Pro Tyr His Val
1               5                   10                  15

Ser Leu Asn Ser Gly Ser Asn Ser Thr Thr Ile Cys Gly Gly Ala Leu
            20                  25                  30

Ile Asn Glu Gln Trp Val Val Thr Ala Gly His Cys Tyr Ser Phe Leu
        35                  40                  45

Pro Leu Asp Lys Ser Arg Ile Gln Val Arg Leu Gly Val His Ser Thr
    50                  55                  60

Gly Ala Leu Glu Gly Asn Glu Gln Ile Ile Asn Ala Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Ser Pro Ala Val Ile Asn Ala His Val Ser Thr Ile
            100                 105                 110

Ser Leu Pro Thr Ala Pro Pro Ala Thr Gly Thr Lys Cys Leu Ile Ser
        115                 120                 125

Gly Trp Gly Asn Ser Lys Asp Pro Tyr Lys Pro Ser Ser Arg Ala Asp

130                 135                 140

Tyr Pro Gly Glu Val Gln Cys Leu Asp Ala Pro Val Leu Ser Gln Thr
145                 150                 155                 160

Lys Cys Glu Ala Ser Tyr Pro Gly Lys Ile Thr Ser Asn Met Phe Cys
                165                 170                 175

Ala Gly Phe Leu Glu Gly Gly Lys Asp Ser Cys Gln Gly Asp Ser Gly
            180                 185                 190

Gly Pro Val Val His Asn Gly Gln Leu Gln Gly Val Val Ser Trp Gly
        195                 200                 205

Glu Gly Cys Ala Gln Lys Asn Lys Pro Gly Val Tyr Thr Asn Val Tyr
    210                 215                 220

Asn Tyr Val Glu Trp Ile Gln Asp Thr Ile Ala Ala Asn Ser
225                 230                 235

<210>  4
<211>  241
<212>  PRT
<213>  artificial

<220>
<223>  variant G

<400>  4

Ile Val Gly Gly Tyr Asp Cys Glu Glu Asn Ser Val Pro Tyr His Val
1               5                   10                  15

Ser Leu Asn Ser Asp Ser Asn Ser Thr Thr Ile Cys Gly Gly Ala Leu
            20                  25                  30

Ile Asn Glu Gln Trp Val Val Thr Ala Gly His Cys Tyr Ser Phe Leu
        35                  40                  45

Pro Leu Asp Lys Ser Arg Ile Gln Val Arg Leu Gly Val His Ser Thr
    50                  55                  60

Gly Ala Leu Glu Gly Asn Glu Gln Ile Ile Asn Ala Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Ser Pro Ala Val Ile Asn Ala His Val Ser Thr Ile
            100                 105                 110

Ser Leu Pro Thr Ala Pro Pro Ala Thr Gly Thr Lys Cys Leu Ile Ser
        115                 120                 125

Gly Trp Gly Asn Ser Lys Asp Pro Tyr Lys Pro Ser Ser Gly Pro Ser
    130                 135                 140

Thr Ala Asp Tyr Pro Gly Glu Val Gln Cys Leu Asp Ala Pro Val Leu
145                 150                 155                 160

Ser Gln Thr Lys Cys Glu Ala Ser Tyr Pro Gly Lys Ile Thr Ser Asn
                165                 170                 175

Met Phe Cys Ala Gly Phe Leu Glu Gly Gly Lys Asp Ser Cys Gln Gly
            180                 185                 190

Asp Ser Gly Gly Pro Val Val His Asn Gly Gln Leu Gln Gly Val Val
        195                 200                 205

Ser Trp Gly Glu Gly Cys Ala Gln Lys Asn Lys Pro Gly Val Tyr Thr
    210                 215                 220

Asn Val Tyr Asn Tyr Val Glu Trp Ile Gln Asp Thr Ile Ala Ala Asn
225                 230                 235                 240

Ser

<210>  5
<211>  241
<212>  PRT
<213>  artificial

<220>
<223>  variant H

<400>  5

Ile Val Gly Gly Tyr Asp Cys Glu Glu Asn Ser Val Pro Tyr His Val
1               5                   10                  15

Ser Leu Asn Ser Val Ser Asn Ser Thr Thr Ile Cys Gly Gly Ala Leu
            20                  25                  30

Ile Asn Glu Gln Trp Val Val Thr Ala Gly His Cys Tyr Ser Phe Leu
        35                  40                  45

Pro Leu Asp Lys Ser Arg Ile Gln Val Arg Leu Gly Val His Ser Thr
    50                  55                  60

Gly Ala Leu Glu Gly Asn Glu Gln Ile Ile Asn Ala Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Thr Pro Ala Val Ile Asn Asp His Val Ser Thr Ile
            100                 105                 110

Ser Leu Pro Thr Ala Pro Pro Ala Pro Gly Thr Lys Cys Leu Ile Ser
        115                 120                 125

Gly Trp Gly Asn Ser Lys Asp Pro Tyr Lys Pro Ser Ser Gly Pro Ser
    130                 135                 140

Thr Ala Asp Tyr Pro Gly Glu Val Gln Cys Leu Asp Ala Pro Val Leu
145                 150                 155                 160

Ser Gln Thr Lys Cys Glu Ala Ser Tyr Pro Gly Lys Ile Thr Ser Asn
                165                 170                 175

Met Phe Cys Ala Gly Phe Leu Glu Gly Gly Lys Asp Ser Cys Gln Gly
            180                 185                 190

Asp Ser Gly Gly Pro Val Val Gln Asn Gly Gln Leu Gln Gly Val Val
        195                 200                 205

Ser Trp Gly Glu Gly Cys Ala Gln Lys Asn Lys Pro Gly Val Tyr Thr
    210                 215                 220

Asn Val Tyr Asn Tyr Val Glu Trp Ile Gln Asp Thr Ile Ala Ala Asn
225                 230                 235                 240

Ser

<210>  6
<211>  241
<212>  PRT
<213>  artificial

<220>
<223>  variant I

<400>  6

Ile Val Gly Gly Tyr Asp Cys Glu Glu Asn Ser Val Pro Tyr His Val
1               5                   10                  15

Ser Leu Asn Ser Val Ser Asn Ser Thr Thr Ile Cys Gly Gly Ala Leu
            20                  25                  30

Ile Asn Glu Gln Trp Val Val Thr Ala Gly His Cys Tyr Ser Phe Leu
        35                  40                  45

Pro Leu Asp Lys Ser Arg Ile Gln Val Arg Leu Gly Met His Ser Thr
    50                  55                  60

Arg Ala Leu Glu Gly Asn Glu Gln Ile Ile Asn Ala Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Thr Pro Ala Val Ile Asn Asp His Val Ser Thr Ile
            100                 105                 110

Ser Leu Pro Thr Ala Pro Pro Ala Ala Gly Thr Lys Cys Leu Ile Ser
        115                 120                 125

Gly Trp Gly Asn Ser Lys Asp Pro Tyr Lys Pro Ser Ser Gly Pro Ser
    130                 135                 140

Thr Ala Asp Tyr Pro Gly Glu Val Gln Cys Leu Asp Ala Pro Val Leu
145                 150                 155                 160

Ser Gln Thr Lys Cys Glu Ala Ser Tyr Pro Gly Lys Ile Thr Ser Asn
                165                 170                 175

Met Phe Cys Ala Gly Phe Leu Glu Gly Gly Lys Asp Thr Cys Gln Gly
            180                 185                 190

Asp Ser Gly Gly Pro Val Val Gln Asn Gly Gln Leu Gln Gly Val Val
        195                 200                 205

Ser Trp Gly Glu Gly Cys Ala Gln Lys Asn Lys Pro Gly Val Tyr Thr
    210                 215                 220

Asn Val Tyr Asn Tyr Val Glu Trp Ile Gln Asp Thr Ile Ala Ala Asn
225                 230                 235                 240

Ser

<210> 7
<211> 241
<212> PRT

<213>  artificial

<220>
<223>  variant J

<400>  7

Ile Val Gly Gly Tyr Asp Cys Glu Glu Asn Thr Val Pro Tyr His Val
1               5                   10                  15

Ser Leu Asn Ser Asp Ser Asn Ser Thr Thr Ile Cys Gly Gly Ala Leu
            20                  25                  30

Ile Asn Glu Gln Trp Val Val Thr Ala Ser His Cys Tyr Ser Phe Leu
        35                  40                  45

Pro Leu Asp Lys Ser Arg Ile Gln Val Arg Leu Gly Met His Ser Thr
    50                  55                  60

Arg Ala Leu Glu Gly Asn Glu Gln Ile Ile Asn Ala Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Thr Pro Ala Val Ile Asn Asp His Val Ser Thr Ile
            100                 105                 110

Ser Leu Pro Thr Ala Pro Pro Ala Ala Gly Thr Met Cys Leu Ile Ser
        115                 120                 125

Gly Trp Gly Asn Ser Lys Asp Pro Tyr Lys Pro Ser Ser Gly Pro Ser
    130                 135                 140

Thr Ala Asp Tyr Pro Gly Glu Val Gln Cys Leu Asp Ala Pro Val Leu
145                 150                 155                 160

Ser Gln Thr Lys Cys Glu Ala Ser Tyr Pro Gly Lys Ile Thr Ser Asn
                165                 170                 175

Met Phe Cys Ala Gly Phe Leu Glu Gly Gly Lys Asp Thr Cys Gln Gly
            180                 185                 190

Asp Ser Gly Gly Pro Ala Val Gln Asn Gly Gln Leu Gln Gly Val Val
        195                 200                 205

Ser Trp Gly Glu Gly Cys Ala Gln Lys Asn Lys Pro Gly Val Tyr Thr
    210                 215                 220

Asn Val Tyr Asn Tyr Val Glu Trp Ile Gln Asp Thr Ile Ala Ala Asn
225                 230                 235                 240

Ser

<210>  8
<211>  241
<212>  PRT
<213>  artificial

<220>
<223>  variant K

<400>  8

Ile Val Gly Gly Tyr Asp Cys Glu Glu Asn Ser Val Pro Tyr His Val
1               5                   10                  15

Ser Leu Asn Ser Asp Ser Asp Ser Thr Thr Ile Cys Gly Gly Ala Leu
            20                  25                  30

Ile Asn Glu Gln Trp Val Val Thr Ala Ser His Cys Tyr Ser Phe Leu
        35                  40                  45

Pro Leu Asp Lys Ser Arg Thr Gln Val Arg Leu Gly Met His Ser Thr
    50                  55                  60

Gly Ala Leu Glu Gly Asn Glu Gln Ile Ile Asp Val Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Thr Pro Ala Val Ile Asn Asp His Val Ser Thr Ile
            100                 105                 110

Ser Leu Pro Thr Ala Pro Pro Ala Ala Gly Thr Met Cys Leu Val Ser
        115                 120                 125

Gly Trp Gly Asn Ser Lys Asp Pro Tyr Lys Pro Ser Ser Gly Pro Pro
    130                 135                 140

Thr Ala Asp Tyr Pro Gly Glu Val Gln Cys Leu Asp Ala Pro Val Leu
145                 150                 155                 160

Ser Gln Thr Lys Cys Glu Ala Ser Tyr Pro Gly Lys Ile Thr Ser Asn
                165                 170                 175

Met Phe Cys Ala Gly Phe Leu Glu Gly Gly Lys Asp Thr Cys Gln Gly

180                 185                 190

Asp Ser Gly Gly Pro Ala Val Gln Asp Gly Gln Leu Gln Gly Val Val
        195                 200                 205

Ser Trp Gly Glu Gly Cys Ala Gln Lys Asn Lys Pro Gly Val Tyr Thr
    210                 215                 220

Asn Val Tyr Asn Tyr Val Glu Trp Ile Arg Asp Thr Ile Ala Ala Asn
225                 230                 235                 240

Ser

<210>  9
<211>  241
<212>  PRT
<213>  artificial

<220>
<223>  variant L

<400>  9

Ile Val Gly Gly Tyr Asp Cys Glu Glu Asn Thr Val Pro Tyr His Val
1               5                   10                  15

Ser Leu Asn Ser Asp Ser Asn Ser Thr Thr Ile Cys Gly Gly Ala Leu
            20                  25                  30

Ile Asn Glu Gln Trp Val Val Thr Ala Ser His Cys Tyr Ser Phe Leu
        35                  40                  45

Pro Leu Asp Lys Ser Arg Ile Gln Val Arg Leu Gly Met His Ser Thr
    50                  55                  60

Arg Ala Leu Glu Gly Asn Glu Gln Ile Ile Asn Ala Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Thr Pro Ala Val Ile Asn Asp His Val Ser Thr Ile
            100                 105                 110

Ser Leu Pro Thr Ala Pro Pro Ala Ala Gly Thr Met Cys Leu Ile Ser
        115                 120                 125

Gly Trp Gly Asn Ser Lys Asp Pro Tyr Lys Pro Ser Ser Gly Pro Ser
    130                 135                 140

Thr Ala Asp Tyr Pro Gly Glu Val Gln Cys Leu Asp Ala Pro Val Leu
145                 150                 155                 160

Ser Gln Thr Lys Cys Glu Ala Ser Tyr Pro Gly Lys Ile Thr Ser Asn
                165                 170                 175

Met Phe Cys Ala Gly Phe Leu Glu Gly Gly Lys Asp Thr Cys Gln Gly
            180                 185                 190

Asp Ser Gly Gly Pro Ala Val Gln Asn Gly Gln Leu Gln Gly Val Val
        195                 200                 205

Ser Trp Gly Glu Gly Cys Ala Gln Lys Asn Lys Pro Gly Val Tyr Thr
    210                 215                 220

Asn Val Tyr Asn Tyr Val Glu Trp Ile Gln Asp Thr Ile Ala Ala Asn
225                 230                 235                 240

Ser

<210>  10
<211>  241
<212>  PRT
<213>  artificial

<220>
<223>  variant M

<400>  10

Ile Val Gly Gly Tyr Asp Cys Glu Glu Asn Ser Val Pro Tyr His Val
1               5                   10                  15

Ser Leu Asn Ser Asp Ser Asp Ser Thr Thr Ile Cys Gly Gly Ala Leu
            20                  25                  30

Ile Asn Glu Gln Trp Val Val Thr Ala Ser His Cys Tyr Ser Phe Leu
        35                  40                  45

Pro Leu Asp Lys Ser Arg Thr Gln Val Arg Leu Gly Met His Ser Thr
    50                  55                  60

Gly Ala Leu Glu Gly Asn Glu Gln Ile Ile Asp Val Ala Lys Ile Ile
65                  70                  75                  80

Arg His Pro Gln Tyr Asp Trp Lys Thr Leu Asn Asn Asp Ile Met Leu
                85                  90                  95

Ile Lys Leu Ser Thr Pro Ala Val Ile Asn Asp His Val Ser Thr Ile
            100                 105                 110

Ser Leu Pro Thr Ala Pro Pro Ala Ala Gly Thr Met Cys Leu Val Ser
        115                 120                 125

Gly Trp Gly Asn Ser Lys Asp Pro Tyr Lys Pro Ser Ser Gly Pro Pro
    130                 135                 140

Thr Ala Asp Tyr Pro Gly Glu Val Gln Cys Leu Asp Ala Pro Val Leu
145                 150                 155                 160

Ser Gln Thr Lys Cys Glu Ala Ser Tyr Pro Gly Lys Ile Thr Ser Asn
                165                 170                 175

Met Phe Cys Ala Gly Phe Leu Glu Gly Gly Lys Asp Thr Cys Gln Gly
            180                 185                 190

Asp Ser Gly Gly Pro Ala Val Gln Asp Gly Gln Leu Gln Gly Val Val
        195                 200                 205

Ser Trp Gly Glu Gly Cys Ala Gln Lys Asn Lys Pro Gly Val Tyr Thr
    210                 215                 220

Asn Val Tyr Asn Tyr Val Glu Trp Ile Arg Asp Thr Ile Ala Ala Asn
225                 230                 235                 240

Ser

<210>  11

<211>  157
<212>  PRT
<213>  homo sapiens

<400>  11

<210>  12
<211>  159
<212>  PRT
<213>  artificial

<220>
<223>  cloned human TNF-alpha

<400>  12

Met Ala His His His His Val Cys Thr Pro Ser Asp Lys Pro Val Ala
1               5                   10                  15

His Val Val Ala Asn Pro Gln Ala Glu Gly Gln Leu Gln Trp Leu Asn
            20                  25                  30

Arg Arg Ala Asn Ala Leu Leu Ala Asn Gly Val Glu Leu Arg Asp Asn
        35                  40                  45

Gln Leu Val Val Pro Ser Glu Gly Leu Tyr Leu Ile Tyr Ser Gln Val
    50                  55                  60

Leu Phe Lys Gly Gln Gly Cys Pro Ser Thr His Val Leu Leu Thr His
65                  70                  75                  80

Thr Ile Ser Arg Ile Ala Val Ser Tyr Gln Thr Lys Val Asn Leu Leu
                85                  90                  95

Ser Ala Ile Lys Ser Pro Cys Gln Arg Glu Thr Pro Glu Gly Ala Glu
            100                 105                 110

Ala Lys Pro Trp Tyr Glu Pro Ile Tyr Leu Gly Gly Val Phe Gln Leu
        115                 120                 125

Glu Lys Gly Asp Arg Leu Ser Ala Glu Ile Asn Arg Pro Asp Tyr Leu
    130                 135                 140

Leu Phe Ala Glu Ser Gly Gln Val Tyr Phe Gly Ile Ile Ala Leu
145                 150                 155

<210>  13
<211>  16
<212>  PRT
<213>  artificial

<220>
<223>  assay peptide

<400>  13

Gly Gln Leu Gln Trp Leu Asn Arg Arg Ala Asn Ala Leu Leu Ala Cys
1               5                   10                  15

<210>  14
<211>  29
<212>  DNA
<213>  artificial

<220>
<223>  primer A

<400>  14
tggcaggagg ggccactcag gcctttgca                                         29

<210>  15
<211>  26
<212>  DNA
<213>  artificial

<220>
<223>  primer B

<400>  15
cacctagtgg cctagtcggc cttagc                                            26

<210>  16
<211>  6
<212>  PRT
<213>  artificial

<220>
<223>  general SDR2 sequence

<220>
<221>  MISC_FEATURE

<222>  (1)..()
<223>  Xaa= small or charged amino acid

<220>
<221>  MISC_FEATURE
<222>  (2)..()
<223>  Xaa= P, N or hydrophobic amino acid

<220>
<221>  MISC_FEATURE
<222>  (3)..()
<223>  Xaa= hydrophobic amino acid

<220>
<221>  MISC_FEATURE
<222>  (4)..()
<223>  Xaa= P, D, N or hydrophobic amino acid

<220>
<221>  MISC_FEATURE
<222>  (5)..()
<223>  Xaa= P, T, R or hydrophobic amino acid

<220>
<221>  MISC_FEATURE
<222>  (6)..()
<223>  Xaa= A, L or polar amino acid

<400>  16

Xaa Xaa Xaa Xaa Xaa Xaa
1               5


<210>  17
<211>  5
<212>  PRT
<213>  artificial

<220>
<223>  general SDR3 sequence


<220>
<221>  MISC_FEATURE
<222>  (1)..()
<223>  Xaa= L, R or small amino acid

<220>
<221>  MISC_FEATURE
<222>  (2)..()
<223>  Xaa= V, T, S, A, M, G or positive amino acid

<220>
<221>  MISC_FEATURE
<222>  (3)..()
<223>  Xaa= D, A, P, T, N, G, E, L, Q, V, S, R

<220>
<221>  MISC_FEATURE
<222>  (4)..()
<223>  Xaa= F, P, S, D, V, Q, T, R, L, I, Y

<220>
<221>  MISC_FEATURE
<222>  (5)..()
<223>  Xaa= peptidic moiety having 1 to 4 arbitrary amino acids

<400>  17

Xaa Xaa Xaa Xaa Xaa
1               5


<210>  18
<211>  4
<212>  PRT
<213>  artificial

<220>
<223>  SDR3 fragment

<400>  18

Arg Asp Pro Tyr
1


<210>  19
<211>  4
<212>  PRT
<213>  artificial

<220>
<223>  SDR3 fragment

<400>  19

Gly Ala Leu Tyr
1


<210>  20
<211>  6
<212>  PRT
<213>  artificial

<220>
<223>  SDR2a

<400>  20

Ala Phe Phe Pro Ala Asp
1               5


<210>  21
<211>  6
<212>  PRT
<213>  artificial

<220>
<223>  SDR2b

<400>  21

Ser Phe Leu Pro Leu Asp
1               5


<210>  22
<211>  5
<212>  PRT
<213>  artificial

<220>
<223>  SDR3a

<400>  22

Ser Lys Asp Pro Tyr
1               5


<210>  23
<211>  8
<212>  PRT
<213>  artificial

<220>
<223>  SDR3b

<400>  23

Ser Lys Gly Pro Arg Asp Pro Tyr
1               5


<210>  24
<211>  8
<212>  PRT
<213>  artificial

<220>
<223>  SDR3c

<400>  24

Ser Lys Asn Pro Gly Ala Leu Tyr
1               5


<210>  25
<211>  224
<212>  PRT
<213>  Homo sapiens

<400>  25

Ile Val Gly Gly Tyr Ile Cys Glu Glu Asn Ser Val Pro Tyr Gln Val
1               5                   10                  15


Ser Leu Asn Ser Gly Tyr His Phe Cys Gly Gly Ser Leu Ile Ser Glu
            20                  25                  30


Gln Trp Val Val Ser Ala Gly His Cys Tyr Lys Ser Arg Ile Gln Val
        35                  40                  45

Arg Leu Gly Glu His Asn Ile Glu Val Leu Glu Gly Asn Glu Gln Phe
    50                  55                  60

Ile Asn Ala Ala Lys Ile Ile Arg His Pro Lys Tyr Asn Ser Arg Thr
65                  70                  75                  80

Leu Asp Asn Asp Ile Leu Leu Ile Lys Leu Ser Ser Pro Ala Val Ile
                85                  90                  95

Asn Ser Arg Val Ser Ala Ile Ser Leu Pro Thr Ala Pro Pro Ala Ala
            100                 105                 110

Gly Thr Glu Ser Leu Ile Ser Gly Trp Gly Asn Thr Leu Ser Ser Gly
        115                 120                 125

Ala Asp Tyr Pro Asp Glu Leu Gln Cys Leu Asp Ala Pro Val Leu Ser
    130                 135                 140

Gln Ala Glu Cys Glu Ala Ser Tyr Pro Gly Lys Ile Thr Asn Asn Met
145                 150                 155                 160

Phe Cys Val Gly Phe Leu Glu Gly Gly Lys Asp Ser Cys Gln Gly Asp
                165                 170                 175

Ser Gly Gly Pro Val Val Ser Asn Gly Glu Leu Gln Gly Ile Val Ser
            180                 185                 190

Trp Gly Tyr Gly Cys Ala Gln Lys Asn Arg Pro Gly Val Tyr Thr Lys
        195                 200                 205

Val Tyr Asn Tyr Val Asp Trp Ile Lys Asp Thr Ile Ala Ala Asn Ser
    210                 215                 220

<210>  26
<211>  224
<212>  PRT
<213>  Homo sapiens

<400>  26

Ile Val Gly Gly Tyr Thr Cys Glu Glu Asn Ser Leu Pro Tyr Gln Val
1               5                   10                  15

Ser Leu Asn Ser Gly Ser His Phe Cys Gly Gly Ser Leu Ile Ser Glu
            20                  25                  30

Gln Trp Val Val Ser Ala Ala His Cys Tyr Lys Thr Arg Ile Gln Val
        35                  40                  45

Arg Leu Gly Glu His Asn Ile Lys Val Leu Glu Gly Asn Glu Gln Phe
    50                  55                  60

Ile Asn Ala Ala Lys Ile Ile Arg His Pro Lys Tyr Asn Arg Asp Thr
65                  70                  75                  80

Leu Asp Asn Asp Ile Met Leu Ile Lys Leu Ser Ser Pro Ala Val Ile
                85                  90                  95

Asn Ala Arg Val Ser Thr Ile Ser Leu Pro Thr Ala Pro Pro Ala Ala
            100                 105                 110

Gly Thr Glu Cys Leu Ile Ser Gly Trp Gly Asn Thr Leu Ser Phe Gly
        115                 120                 125

Ala Asp Tyr Pro Asp Glu Leu Lys Cys Leu Asp Ala Pro Val Leu Thr
    130                 135                 140

Gln Ala Glu Cys Lys Ala Ser Tyr Pro Gly Lys Ile Thr Asn Ser Met
145                 150                 155                 160

Phe Cys Val Gly Phe Leu Glu Gly Gly Lys Asp Ser Cys Gln Arg Asp
                165                 170                 175

Ser Gly Gly Pro Val Val Cys Asn Gly Gln Leu Gln Gly Val Val Ser
            180                 185                 190

Trp Gly His Gly Cys Ala Trp Lys Asn Arg Pro Gly Val Tyr Thr Lys
        195                 200                 205

Val Tyr Asn Tyr Val Asp Trp Ile Lys Asp Thr Ile Ala Ala Asn Ser
    210                 215                 220

<210>  27
<211>  50
<212>  DNA
<213>  artificial

<220>
<223>  primer AF

<400>  27
ctggcaggag gggccactca ggcctttgca atcgttgggg gctacaactg                50

<210>  28
<211>  23
<212>  DNA
<213>  artificial

<220>
<223>  primer AR

<400>  28
gtagcagtgg cccgcggata cca                                              23


<210>  29
<211>  18
<212>  DNA
<213>  artificial

<220>
<223>  primer BF

<400>  29
aagtcccgga tccaggtg                                                    18


<210>  30
<211>  19
<212>  DNA
<213>  artificial

<220>
<223>  primer BR

<400>  30
gttgccccag ccagagatg                                                   19


<210>  31
<211>  21
<212>  DNA
<213>  artificial

<220>
<223>  primer CF

<400>  31
gtcggcgcca gagctagcag t                                                21


<210>  32
<211>  44
<212>  DNA
<213>  artificial

<220>
<223>  primer CR

<400>  32
acacctagtg gcctagtcgg ccttagctat tggcagctat ggtg                       44


<210>  33
<211>  59
<212>  DNA
<213>  artificial

<220>
<223>  primer DF

<220>
<221>  misc_feature
<222>  (24)..(40)
<223>  n= a, t, c, g

<220>
<221>  misc_feature
<222>  (26)..(41)
<223>  b= t, c, g

<400>  33
tggtatccgc gggccactgc tacnnbnnbn nbnnbnnbnn baagtcccgg atccaggtg      59


<210>  34
<211>  52
<212>  DNA
<213>  artificial

<220>
<223>  primer DR


<220>
<221>  misc_feature
<222>  (19)..(31)
<223>  v= g, c, a

<220>
<221>  misc_feature
<222>  (20)..(33)
<223>  n= a, t, c, g

<400>  34
ggcgccagag ctagcagtvn nvnnvnnvnn vnngttgccc cagccagaga tg            52

## Description

The present invention is directed to specific proteases, fragments and derivatives thereof that specifically inactivate tumour necrosis factor-alpha, methods for their preparation, pharmaceutical and diagnostic compositions comprising such a protease. The proteases of the invention are useful for the treatment of clinical conditions resulting from the detrimental activity of tumour necrosis factor. The invention further relates to nucleotide sequences thereof encoding the protease, as well as vectors and hosts containing such sequences.

### Background of the Invention

The tumour necrosis factor-alpha (TNF-alpha, TNFα) is a homotrimeric protein (Eck et al., Journal of Biological Chemistry, 264(29):17595-17605 (1989)) expressed primarily by cells of the immune system, namely macrophages and monocytes. Exposure of host cells to antigen, cytokines, infectious pathogens, or stress signals initiate signalling pathways that lead to the activation of transcription factors in the nucleus that orchestrate the tightly regulated induction of TNF-alpha expression. Each subunit is initially translated as a 26 kDa transmembrane precursor protein that upon cleavage at a site proximal to the transmembrane domain is released as a 17 kDa circulating soluble protein. TNF-alpha is a pleiotropic, multifunctional cytokine that mediates key roles in acute and chronic inflammation, cell growth, antiviral activity, vasculature and extracellular matrix remodelling, anti-tumour responses, pathogenesis of many infections and bone resorption, among others. The primary biological function of TNF-alpha is that of a pro-inflammatory cytokine (Tracey et al., Annual Review in Medicine, 45:491-503 (1994)).

TNF-alpha exerts its activity by binding to two structurally distinct TNF receptors, TNF-R1 and TNF-R2, on effector cells, triggering signalling cascades that include TRAF-2 and the more downstream kinases IKK, p38, ERK, and JNK. These signals in turn mediate the activation of the target cell effector functions, namely cytokine expression such as Interleukin (IL)-6, granulocyte-macrophage colony-stimulating factor (GM-CSF) and IL-8, cellular proliferation and apoptosis among others. The concurrent expression of other inflammatory cytokines, namely IL-1, and IL-6, also contribute and synergize with TNF-alpha in the onset of clinical pathologies. In addition to the activation of pro-inflammatory cascades, TNF-alpha also possesses anti-tumour and pro-apoptotic activities which it mediates via distinct mechanisms, namely the activation of caspase pathways.

The relevance of human TNF-alpha (hTNFα) as a therapeutic target has been demonstrated experimentally by intensive research of its role in several immune system pathways. Consequently, it has been implicated in the pathophysiology of a number of diseases, including sepsis and trauma-induced shock, allograft rejection, rheumatoid arthritis (RA), inflammatory bowl disease (IBD), Crohn's disease (CD), psoriasis and many other chronic and acute inflammatory diseases. Namely, a key role for hTNFα in the initiation and/or perpetuation of the inflammatory processes in RA has been confirmed by several studies in pharmacologically relevant experimental animal models and validated clinically in RA, CD and psoriatic patients treated with biologicals that neutralize its activity. These studies elucidated the role of the cytokine in the induction of other pro-inflammatory cytokine cascades by macrophages and fibroblasts, which consequently lead to chronic inflammation. Therefore, an anti-hTNFα treatment is expected to affect a broad range of physiological networks.

Significant efforts have been applied to the development of medicinal compounds that specifically modulate the activity of hTNFα in physiologically relevant disease settings. These aim to diminish the synthesis and release of hTNFα, to bind to hTNFα or otherwise interfere with its cellular receptor binding or to modify downstream intracellular signalling pathways. Some of these approaches resulted in the development of immunoglobulin-derived biologicals for the inhibition of hTNFα activity and which received market approval for the treatment of several inflammatory diseases.

Etanercept (Enbrel^{®}) represents one such approach to targeting hTNFα for neutralization of its disease inducing activity. This hTNFα antagonist consists of two recombinant copies of the human soluble TNF-receptor II (TNF-R2, p75) fused to the Fc portion of human IgG1. The Fc component contains the C_{H}2 domain, the C_{H}3 domain and hinge region, but not the C_{H}1 domain of IgG1. Etanercept is produced by recombinant methods in a Chinese hamster ovary (CHO) mammalian cell expression system. Etanercept binds both hTNFα and human lymphotoxin-alpha but it does not fix complement or lyse cells. The terminal half-life is approximately three days, which is half that of infliximab.

Infliximab (Remicade^{®}) is a chimeric monoclonal antibody in which the murine constant region has been replaced by human equivalent sequences. It neutralizes the biological activity of hTNFα by binding to both the soluble and transmembrane forms of hTNFα and inhibits binding of hTNFα to its receptors (Reimold, A.M., Current Drug Targets- Inflammation & Allergy, 1:377-392 (2002)). In addition to receptor binding antagonism, infliximab is also capable of directly inducing apoptosis of membrane hTNFα expressing cells.

Adalimumab (Humira^{®}) is a fully human antibody generated by phage-display technology, resulting in an antibody with human heavy- and light-chain variable regions, and human immunoglobulin IgG1-κ- constant regions. Adalimumab also binds to hTNFα and blocks its interaction with the TNF receptor and lyse hTNFα - expressing cells in the presence of complement. The half-life of Adalimumab is 10-20 days, which is longer than non-human antibodies (Bain, B. and Brazil, M., Nature Reviews, 2:693-694 (2003)). As with infliximab, Adalimumab has been shown to directly induce apoptosis of membrane bound hTNFα.

The disadvantage of immunoglobulin derived therapeutics is that they directly interfere with the function of immune effector cells, namely those expressing Fc-receptors such as macrophages which play a crucial role in innate immunity. This can cause immune related adverse events seen with antibody based therapies, namely induction of cytokine release syndrome. There is also a potential risk of infectious diseases such as tuberculosis due to its interference with the innate immune system.

Additionally, hTNFα antibodies have been reported to cause the formation of anti-dsDNA antibodies (ADA), and after repeated treatment the cumulative ADA incidence can be as high as 50%. Demyelinising disease and aplastic anaemia have been reported in a small number of Infliximab and Etanercept treated patients (van Deventer, S.J.H., Gut, 51:362-363 (2002); Kuruvilla, J., Eur J Haematol., 71(5):396-8 (2003)). A likely cause is induction of apoptotic cell death via engagement of the membrane form of hTNFα or Fc-receptors. Upon cell lysis, acute exposure to genomic DNA induces an immune response.

Target-specific proteases as described in WO 2004/113521 and WO 2004/113522 represent a new generation of biopharmaceuticals. The "New Biological Entity" technology (NBE^{®}) provides a platform for the provision of proteases with novel functions that do not exist in the components used as source material of such proteins. The NBE^{®} platform is characterised by the re-engineering of a protease scaffold retaining its basic catalytic function and combining it with one or more specificity determining regions (SDR^{®}) thus rendering it specific for a particular substrate molecule. The one or more SDRs are located at sites within the protein scaffold that enables the resulting engineered protein to discriminate between at least one target substrate and one or more different substrates, which would not have been discriminated by the unmodified protease. A clear benefit of target-specific proteases is the irreversible inactivation of the target substrate by hydrolysis whereas target neutralization by binding of therapeutic antibodies is reversible. Furthermore, target-specific proteases have a higher efficiency due to the catalytic substrate turnover. Therefore, one protease molecule can inactivate a very high number of hTNFα molecules while antibodies exhibit only a stoichiometric neutralization by antigen-antibody binding.

As the public health systems are increasingly forced to reduce their expenditure in the future, costs for a particular treatment are considered critical for the development of an innovative drug to provide the public with new effective treatments. Antibody-based therapeutics are produced in mammalian cell culture that is a costly process.

### Summary of the Invention

Existing therapeutics targeting hTNFα encounter several disadvantages. It is the objective of the present invention to provide a hTNFα targeting protease, preferentially expressed in a microbial expression system. In particular, the invention provides proteases with a scaffold capable of hydrolyzing the protein hTNFα and one or more specificity determining regions (SDR^{®}) to discriminate hTNFα from other potential protein substrates. Furthermore, it is an objective of the present invention to provide pharmaceutical or diagnostic compositions comprising such protease variants. Another objective of the present invention is to provide nucleotide sequences or fragments thereof encoding the protease, as well as vectors and hosts containing such sequences.

This problem has been solved by the embodiments of the invention specified in the description below and in the claims. The present invention is thus directed to
(1) a protease capable of inactivating human tumour necrosis factor-alpha (hTNFα) of SEQ ID NO: 10
   (a) having a primary structure based on a scaffold derived from wild-type human cationic trypsin having the amino acid sequence shown in SEQ ID NO:1 or from a functional and/or structural equivalent thereof, and
   (b)having a first, second and third specificity determining region (SDR) located between residues 21 and 22, 42 and 43, and 123 and 124 in the scaffold, respectively, wherein the numbering refers to the wild-human cationic trypsin as shown in SEQ ID NO: 1,
      said first SDR having a length of up to 8 amino acid residues and comprising the structure -X₁-X₂-X₃-, wherein X₁, X₂ and X₃ are independently selected from hydrophilic amino acid residues,
      said second SDR having the structure -X₄-X₅-X₆-X₇-X₈-X₉- (SEQ ID NO: 16), wherein X₄ is a small or charged amino acid residue, X₅ is P, N or a hydrophobic amino acid residue, X₆ is a hydrophobic amino acid residue, X₇ is P, D, N or a hydrophobic amino acid residue, X₈ is P, T, R or a hydrophobic amino acid residue, and X₉ is A, L or a polar amino acid residue,
      said third SDR having the structure -X₁₀-X₁₁-X₁₂-X₁₃-X₁₄- (SEQ ID NO: 17), wherein X₁₀ is L, R or a small amino acid residue, X₁₁ is V, T, S, A, M, G or a positive amino acid residue, X₁₂ is selected from D, A, P, T, N, G, E, L, Q, V, S and R, X₁₃ is selected from F, P, S, D, V, Q, T, R, L, I and Y, and X₁₄ represents a peptidic moiety having one to four arbitrary amino acid residues,
   and fragments, regions and derivatives thereof that inactivate hTNFα;
(2) a nucleotide sequence encoding the protease according to (1) above;
(3) a vector comprising the nucleotide sequence of (2) above;
(4) a host cell, cell culture, tissue culture or non-human organism being transformed/transfected with the vector of (3) above and/or containing the nucleotide sequence of (2) above;
(5) a method for the preparation of the protease of (1) above, which comprises culturing host cells of (4) above and isolating the protease from the culture;
(6) a pharmaceutical or diagnostic composition comprising the protease of (1) above;
(7) the use of the protease of (1) above for preparing a medicament for the treatment of clinical conditions resulting from the detrimental activity of tumour necrosis factor; and
(8) a method for the treatment of clinical conditions resulting from the detrimental activity of tumour necrosis factor in a patient which comprises administering the patient a suitable amount of the protease of (1) above.

### Brief Description of the Figures

The following figures are provided in order to explain further the present invention in supplement to the detailed description.
Figure 1 shows a mass spectrum of the cleavage product of hTNFα verifying the targeted cleavage site.
Figure 2 deconvoluted mass spectrum of the cleavage product of hTNFα verifying the targeted cleavage site.
Figure 3 demonstrates the progression in specific activity of a panel of variants.
Figure 4 demonstrates that pegylation does not abrogate the activity of variant E.
Figure 5 demonstrates the specificity of different variants in a biochemical assay based on substrate competition
Figure 6 demonstrates the specificity of unpegylated variant E on different human serum proteins in comparison to the unspecific original trypsin scaffold.
Figure 7 demonstrates the activity of a pegylated variant of the protease in a human TNFα transgenic mouse model of polyarthritis. In this particular study, treatment was initiated after the onset of clinical expression (joint inflammation).
Figure 8 demonstration of the selectivity of variant G that does not cleave membrane bound TNFα.
Figure 9 shows an alignment between human trypsin variants. TRY1_HUMAN is human cationic trypsin (SEQ ID NO:1), TRY2_HUMAN is human anionic trypsin (trypsin-2 precursor; SEQ ID NO:25) and TRY3_HUMAN is human mesotrypsin (trypsin-3 precursor; SEQ ID NO:26)

### Definitions

In the framework of the present invention the following terms and definitions are used.

The term "protease" means any protein molecule catalyzing the hydrolysis of peptide bonds. It includes naturally-occurring proteolytic enzymes, as well as protease variants obtained by site-directed or random mutagenesis, insertion, deletion, recombination and/or any other protein engineering method, that leads to novel proteases that differ in their amino acid sequence, physical characteristics and functional properties from the parent protease. It also comprises any fragment of a proteolytic enzyme, any molecular complex, conjugated protease or fusion protein comprising one of the aforementioned proteases.

The term "TNF-alpha specific protease" describes a protein molecule catalyzing specifically the hydrolysis of human TNF-alpha. It is a non-naturally occurring proteolytic enzyme obtained by site-directed or random mutagenesis, insertion, deletion, recombination and/or any other protein engineering method, that leads to a TNF-alpha specific protease that differ in the amino acid sequence by insertion, deletion or substitution of one or more amino acids, physical characteristics and functional properties from the parent protease. It also comprises any fragment of a proteolytic enzyme, any molecular complex, conjugated protease or fusion protein comprising one of the aforementioned protease.

The term "human TNF-alpha" (also abbreviated as hTNFα) refers to the cytokine with the structure and biological function as described further in for example (Tracey et al. (1994) Tumor necrosis factor: a pleiotropic cytokine and therapeutic target; Annual Review in Medicine, 45:491-503). Its definition includes the endogenously expressed secreted soluble cytokine or its cell surface expression in a transmembrane form. Additionally, the term also includes recombinantly expressed and purified protein following standard procedures or purchased commercially from suppliers.

By "pharmacokinetics" means the study of the way the hTNFα specific protease behaves in the body after administration. It assesses the ADME (absorption, distribution, metabolism and excretion) processes by examining the time course of drug concentration profiles in readily accessible body fluids such as blood, plasma, serum or urine. The term "pharmacodynamics" refers to the relationship between protease concentrations in e.g. plasma or at the effect site with the type and magnitude of pharmacological effects of the protease.

The term "terminal half-life" or more simply "half-life" is a parameter of pharmacokinetics and indicates the time necessary for the concentrations of detectable drug in any given body fluid, for example blood serum, to decrease by 50 percent.

The term "efficacy" relates to the concentration of a compound needed to induce a certain degree of a physiological response. A compound X is said to possess higher efficiency than a comparator compound Y, if the concentration of X needed to induce the same response as Y is lower.

The term "substrate" or "peptide substrate" means any peptide, oligopeptide, or protein molecule of any amino acid composition, sequence or length, that contains a peptide bond that can be hydrolyzed catalytically by a protease. The peptide bond that is hydrolyzed is referred to as the "cleavage site".

The term "specificity" means the ability of an enzyme to selectively recognize and preferentially convert certain unique substrates (or targets) while leaving other potential substrates unconverted. For example, proteases that act selectively on a single peptide or protein among all possible peptide or protein substrates have a "high specificity". Proteases that accept almost any peptide or protein substrate have a "low specificity". Proteases with very low specificity are also referred to as "unspecific proteases". The term "defined specificity" refers to a certain type of specificity, i.e. to a certain target substrate or a set of certain target substrates that are preferentially converted versus other substrates.

The term "target" or "pharmaceutical target" refers to the peptide or protein entity that is preferentially cleaved by the protease. In most cases the "target" is causally involved in the onset, progression and/or maintenance of the disease pathology.

The term "protein scaffold" or "scaffold protein" refers to a variety of primary, secondary and tertiary polypeptide structures. Preferably, the scaffold is a protease structure capable of hydrolyzing a specific site in a protein target that leads to activation or inactivation of the protein target.

The term "mutation" refers to the substitution or replacement of single or multiple nucleotide triplets, insertions or deletions of one or more codons, homologous or heterologous recombination between different genes, fusion of additional coding sequences at either end of the encoding sequence, or insertion of additional encoding sequences or any combination of these methods, which result in a polynucleic acid sequence encoding the desired protein. Thus, the term "mutations" also refers to all of the changes in the polypeptide sequence encoded by the polynucleic acid sequence modified by one or more of the above described changes.

Amino acid residues are abbreviated according to the following Table 1 either in one- or in three-letter code.

**Table 1: Amino acid abbreviations**

| Abbreviations | | Amino acid |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |

Mutations are described by use of the following nomenclature: amino acid residue in the protein scaffold; position; substituted amino acid residue(s). According to this nomenclature, the substitution of, for instance, an alanine residue for a glycine residue at position 20 is indicated as Ala20Gly or A20G. The deletion of alanine in the same position is shown as Ala20* or A20*. The insertion of an additional amino acid residue (e.g. a glycine) is indicated as Ala20AlaGly or A20AG. The deletion of a consecutive stretch of amino acid residues (e.g. between alanine at position 20 and glycine at position 21) is indicated as Δ(Ala20-Gly21) or Δ(A20-G21). When a TNF-alpha specific protease contains a deletion in comparison to the scaffold protein used for numbering, an insertion in such a position (e.g. an alanine in the deleted position 20) is indicated as *20Ala or *20A. Multiple mutations are separated by a plus sign or a slash. For example, two mutations in positions 20 and 21 substituting alanine and glutamic acid for glycine and serine, respectively, are indicated as A20G+E21S or A20G/E21S. When an amino acid residue at a given position is substituted with two or more alternative amino acid residues a comma or a slash separates these residues. For example, substitution of alanine at position 30 with either glycine or glutamic acid is indicated as A20G,E or A20G/E, or A20G, A20E. When a position suitable for modification is identified herein without any specific modification being suggested, it is to be understood that any amino acid residue may be substituted for the amino acid residue present in the position. Thus, for instance, when a modification of an alanine in position 20 is mentioned but not specified, it is to be understood that the alanine may be deleted or substituted for any other amino acid residue (i.e. any one of R,N,D,C,Q,E,G,H,I,L,K,M,F,P,S,T,W,Y,V).

The term "conservative mutation" refers to an amino acid mutation that a person skilled in the art would consider to be conservative to a first mutation. "Conservative" in this context means a similar amino acid in terms of the amino acid characteristics. If, for example, a mutation leads at a specific position to a substitution of a non-aliphatic amino acid residue (e.g. Ser) with an aliphatic amino acid residue (e.g. Leu) then a substitution at the same position with a different aliphatic amino acid (e.g. Ile or Val) is referred to as a conservative mutation. Further amino acid characteristics include size of the residue, hydrophobicity, polarity, charge, pK-value, and other amino acid characteristics known in the art. Accordingly, a conservative mutation may include substitution such as basic for basic, acidic for acidic, polar for polar etc. The sets of amino acids thus derived are likely to be conserved for structural reasons. These sets can be described in the form of a Venn diagram (Livingstone, C.D. and Barton, G.J., Comput. Appl. Biosci. 9: 745-756 (1993); Taylor, W.R., J. Theor. Biol. 119; 205-218 (1986)). Conservative substitutions may be made, for example, according to the table below that describes a generally accepted Venn diagram grouping of amino acids.

**Table 2: Venn diagram grouping amino acids**

| Set | | Sub-set | |
|---|---|---|---|
| Hydrophobic | F W Y H K M I L V A G C | Aromatic | F W Y H |
| | | Aliphatic | I L V |
| Polar | W Y H K R E D C S T N Q | Charged | H K R E D |
| | | Positively charged | H K R |
| | | Negatively charged | E D |
| Small | V C A G S P T N D | Tiny | A G S |

The term "peptide sequence" refers to any sequence of one or more amino acids that is part of the scaffold protein, deleted form the scaffold protein, inserted in the protein scaffold, substituted with the protein scaffold or combined with the protein scaffold. Insertion, substitution or combination of peptide sequences with the protein scaffold are generated by insertion, substitution or combination of single or multiple nucleotide triplets into or with a polynucleotide encoding the protein scaffold. The term "synthetic" in combination with the term "peptide sequence" refers to stretches of peptide sequence(s) that are not present in the protein scaffold at the position at which the peptide sequences are inserted or substituted or with which they are combined.

The term "SDR^{®}" or "Specificity Determining Region" refers to a synthetic peptide sequence that enhances specificity for the target when combined with the protein scaffold at sites that enable the resulting proteases to discriminate between the target substrate and one or more other substrates. Such sites are termed "SDR^{®} sites".

By "conjugate" or "conjugated protease" is meant a composite or chimeric molecule formed by the covalent attachment of a protease variant to one or more pharmaceutically acceptable non-polypeptide or polypeptide moieties to improve the pharmacokinetic and pharmacodynamic properties of the protease, e.g. increase terminal half-life in physiological fluids such as blood.

"Covalent attachment" means that the protease variant and the non-polypeptide or polypeptide moiety are either directly joined covalently to one another, or are alternatively indirectly joined covalently to one another through an intervening moiety or moieties, such as a bridge, spacer, or linkage moiety or moieties. Depending on the attachment chemistry applied, the non-polypeptide or polypeptide moiety may be covalently linked in a specific way to a defined attachment group or in a statistical way to a number of attachment groups. Preferably, the conjugation does not significantly alter the specific activity or specificity of the protease and the conjugated protease variant is soluble at relevant concentrations and conditions, i.e. soluble in physiological fluids such as blood. Polymer molecules, lipophilic compounds, sugar moieties or organic derivatising agents are only a few examples for such non-polypeptide moieties. They may or may not have there own biological activity. The non-polypeptide moiety is linked to the protease variant through an attachment group of the variant.

The term "catalytic activity" or "activity" describes quantitatively the conversion of a given substrate under defined reaction conditions. The term "relative activity" is defined as the ratio of the catalytic activity of the enzyme under a certain set of conditions to the catalytic activity under a different set of conditions. Therefore the relative activity aᵢ is given by aᵢ=vᵢ/v₀. and aᵢ*100 is the relative activity in percent.

The term "inhibitor" describes all substances that, when present in the reaction mixture, physically interact with the protease and decrease its catalytic activity compared to the activity in the absence of the substance when all other parameters and concentrations are kept constant. A protease variant is termed "inhibitor insensitive" when the relative activity in the presence of a given amount of inhibitor is higher than for a comparative protease variant.

The term "library of protease variants" describes a mixture of proteases, whereby every single artificial protease is encoded by a different polynucleotide sequence. The term "gene library" indicates a library of polynucleotides that encodes the library of protease variants.

The term "composition" or "pharmaceutical composition" refers to compositions comprising the TNF-alpha specific protease. The compositions are prepared by mixing the TNF-alpha specific protease with any pharmaceutically acceptable component, such as, for example, a carrier, a medicinal agent, an adjuvant, a diluent, and the like, as well as combinations thereof.

The term "vehicle" refers to a diluent, adjuvant, excipient, or carrier with which a composition of the invention is administered.

As used herein, two or more DNA coding sequences are said to be "joined" or "fused" when, as a result of in-frame fusions between the DNA coding sequences, the DNA coding sequences are translated into a fusion polypeptide. A "fusion protein" comprises a protease operatively linked to a second peptide or protein.

### Detailed description of the invention

As set forth above, the present invention is directed to protease variants capable of inactivating human tumour necrosis factor-alpha (hTNF-α) by hydrolysis. These variants have been generated by procedures described in WO 2004/113521 and

WO 2004/113522. In short, the NBE^{®} platform is characterised by taking a protease scaffold with the basic enzymatic functionality and capable of catalyzing a chemical reaction on a substrate and combining it with one or more specificity determining regions (SDR^{®}). The one or more SDRs are located at sites in the protein scaffold that enables the resulting engineered protein to discriminate between at least one target substrate and one or more different substrates, which would not have been discriminated by the unmodified protease. As a protease scaffold any protease can serve, however, preferably human proteases are used and more preferred human cationic trypsin is chosen as a scaffold.

### Description of variants

The primary structure of the proteases variants of the invention is formed by a human cationic trypsin scaffold having the amino acid sequence shown in SEQ ID NO:1 or from a functional and/or structural equivalent thereof (such as human anionic trypsin und human mesotrypsin shown in SEQ ID NOs:25 and 26, respectively) and comprising at least three SDRs located in the scaffold, namely the first, second and third SDR mentioned above. Said scaffold may contain further SDRs and/or one or more additional amino acid substitutions.

It is preferred that the scaffold has a fourth SDR having a length of up to 8, preferably 2 to 5 amino acid residues located between residues 128 and 129 of the scaffold, wherein the numbering refers to the wild-type human cationic trypsin as shown in SEQ ID NO:1. Preferably said fourth SDR comprises at least one P and/or one T residue.

In a preferred embodiment, protease variants having one or more of the following SDRs or a combination of the following SDRs are provided:
The first SDR^{®} has a length of up to 5 amino acid residues and/or comprises at least two serine residues. Preferably X₁ and X₃ within the structure -X₁-X₂-X₃- are serine residues, most preferably the sequence of said first SDR is SNS or SDS.
In the general structure -X₄-X₅-X₆-X₇-X₈-X₉- of the second SDR X₄ is a tiny amino acid residue, X₅ is selected from F, A, I, L, P and N, X₆ is selected from F, L, V, W, A, I and G, X₇ is selected from P, M, V, G, D and N, X₈ is selected from A, L, V, F, P, T, R, Y and G, and/or X₉ is selected from D, S, N, T, A, L and E. Preferably X₄ is A or S, X₅ is F or L, X₆ is F, L or G, X₇ is P or N, X₈ is A, L, V or G, and/or X₉ is D or E.
In the general structure -X₁₀-X₁₁-X₁₂-X₁₃-X₁₄- of the third SDR X₁₀ is selected from A, G, S, T, V, R and L, X₁₁ is selected from K, R, S and T, X₁₂ is selected from D, G, L and N, X₁₃ is selected from P, F and I, and/or X₁₄ is an aromatic amino acid residue, L, G, E, S, T or K, or represents a peptide having four amino acid residues, in which peptide the first and second residues are independently selected from charged and small amino acid residues, the third residue is a small or hydrophobic amino acid residue and the fourth residue is a hydrophobic amino acid residue. Preferably X₁₀ is A, G, S, or L, X₁₁ is K or R, X₁₂ is D, G or N, X₁₃ is P, and/or X₁₄ is Y, W, RDPY (SEQ ID NO: 18) or GALY (SEQ ID NO:19).
The fourth SDR has the general structure -X₁₅-X₁₆-X₁₇-, wherein X₁₅ is P, X₁₆ is an arbitrary amino acid residue and X₁₇ is T. Preferably the sequence of said fourth SDR is PST or PPT.

In an even more preferred embodiment, protease variants having one or more of the following SDRs or a combination of the following SDRs are provided:
The sequence of said first SDR is SNS or SDS.
In the general structure -X₄-X₅-X₆-X₇-X₈-X₉- of the second SDR X₄ is A or S, X₅ is F, X₆ is F or L, X₇ is P, X₈ is A or L, and X₉ is D, Preferably the second SDR has the sequence AFFPAD (SEQ ID NO:20) or SFLPLD (SEQ ID NO:21).
In the general structure -X₁₀-X₁₁-X₁₂-X₁₃-X₁₄- of the third SDR X₁₀ is S, X₁₁ is K, X₁₂ is D, G, or N, X₁₃ is P, and X₁₄ is Y, RDPY (SEQ ID NO: 18) or GALY (SEQ ID NO:19). Preferably the third SDR has the sequence SKDPY (SEQ ID NO:22), SKGPRDPY (SEQ ID NO:23), or SKNPGALY (SEQ ID NO:24).

The sequence of said fourth SDR is PST or PPT.

In addition to the insertions of the SDRs as defined herein before, the proteases of the invention may optionally comprise one or more of the following amino acid substitutions:
G at position 21 is preferably substituted by A, D, S or V, more preferably D or V
Y at position 22 is preferably substituted by T, H, Q, S, W, G or A, more preferably by T or H;
H at position 23 is preferably substituted by T, N, G, D, R or Y, more preferably by T or N;
F at position 24 is preferably substituted by I, V, Q, T, L or A, more preferably by I or V;
S at position 28 is preferably substituted by A;
S at position 37 is preferably substituted by T;
I at position 46 is preferably substituted by V, N, L or T, more preferably by V;
E at position 52 is preferably substituted by V or M, more preferably by V;
N at position 54 is preferably substituted by S;
I at position 55 is preferably substituted by T, N or R, more preferably by T or N;
F at position 64 is preferably substituted by I or T, more preferably by I;
R at position 78 is preferably substituted by W;
S at position 92 is preferably substituted by T;
R at position 93 is preferably substituted by P;
A at position 98 is preferably substituted by D;
R at position 99 is preferably substituted by H;
T at position 112 is preferably substituted by A or P, more preferably by A;
K at position 115 is preferably substituted by M;
A at position 125 is preferably substituted by P or S, more preferably by P;
G at position 128 is preferably substituted by R, K or T, more preferably by R;
Y at position 131 is preferably substituted by F, N or H, more preferably by F;
D at position 133 is preferably substituted by G;
V at position 163 is preferably substituted by A;
S at position 172 is preferably substituted by T;
Q at position 174 is preferably substituted by R;
C at position 183 is preferably substituted by H, Q or R, preferably by H; and/or
D at position 195 is preferably substituted by E (numbering refers to human cationic trypsin as shown in SEQ ID NO:1).

Particularly preferred embodiments of the invention provide TNF-alpha specific proteases based on the human cationic trypsin scaffold with one or more of the above detailed insertions in combination with one or more of the listed substitutions. Specific variants are given in SEQ ID NO:2, SEQ ID NO:3, SEQ ID NO:4, SEQ ID NO:5, SEQ ID NO:6, SEQ ID NO:7, SEQ ID NO:8, SEQ ID NO:9 or SEQ ID NO:10.

### Properties of variants

Preferably, said proteases are capable of specifically inactivating human TNF-alpha of the SEQ ID NO:10, that is, proteolytic cleavage of hTNF-alpha by the protease variant renders hTNF-alpha incapable of exerting its biologic effects. More preferably, said protease variants, a fusion construct of said protease variants or conjugated protease variants are capable of hydrolysing the peptide bonds between positions 31/32 or 32/33 in hTNF-alpha.

In a preferred embodiment of the invention, variants are selective for either soluble or transmembrane human TNF-alpha and in a more preferred embodiment they are selective for soluble TNF-alpha and do not inactivate transmembrane TNF-alpha. Since the action of transmembrane TNF-alpha has been associated with beneficial effects with respect to resistance to bacterial infection and anti-inflammatory effects. The selectivity of variants of the invention provides them with an advantage over currently marketed antibodies that neutralize both soluble and transmembrane TNF-alpha.

The variants of the present invention have a reduced inhibitor sensitivity compared to the parent protease scaffold. In a more preferred embodiment the protease variant is sufficiently insensitive against inhibitors naturally present in the body compartments where its activity is desired, leading to a residual activity that results in efficacy in the intended application. Body compartments where activity may be desired comprise, but are not limited to blood, synovial fluid, interstitial fluid, mucosal fluid, peritoneal fluid, extracellular matrix, the eye, cerebrospinal fluid, the brain, epidermal tissue, different organs as well as epithelial and mucosal surfaces of the body and the intracellular space including cytoplasm or cellular organelles such as lysosomes, endosomes, endoplasmic reticulum, Golgi apparatus, nucleus and mitochondria. In a most preferred embodiment of the invention the protease variant is insensitive to protease inhibitors present in human blood or synovial fluid.

### Isolation of genes encoding for TNF-alpha specific proteases

In a preferred embodiment of the invention, protease variants with the desired properties in terms of specificity, activity, inhibitor insensitivity or any other property are identified in a screening process as described in WO 2004/113521 A1 and WO 2004/113522 A1. The result of the screening process is a culture of a clone of the organism expressing the protease variant of interest. From this culture deoxyribonucleic acid (DNA) sequence coding for said protease can be extracted by standard molecular cloning techniques known to anyone skilled in the art (e.g. Sambrook, J.F, Fritsch, E.F., Maniatis, T., Cold Spring Harbor Laboratory Press, Second Edition, New York (1989)). Isolation and cloning of the gene into suitable vectors allows the determination of the sequence of the deoxyribonucleic acid and thereby the amino acid sequence of the encoded protease by standard techniques.

In order to express the TNF-alpha specific protease, the DNA encoding such TNF-alpha specific protease is ligated into a suitable expression vector by standard molecular cloning techniques (e.g. Sambrook, J.F, Fritsch, E.F., Maniatis, T., Cold Spring Harbor Laboratory Press, Second Edition, New York (1989)). The vector is introduced in a suitable expression host cell that expresses the corresponding TNF-alpha specific protease. Particularly suitable expression hosts are bacterial expression hosts such as Escherichia coli, Pseudomonas fluorescence or Bacillus subtilis, or yeast expression hosts such as Saccharomyces cerevisiae, Kluveromyces lactis, Hansenula polymorpha or Pichia pastoris, other fungal expression hosts such as Aspergillus niger or Trichoderma reesei or mammalian expression hosts such as mouse (e.g., NS0), Chinese Hamster Ovary (CHO) or Baby Hamster Kidney (BHK) cell lines, transgenic mammalian systems such as rabbit, goat or cattle, other eukaryotic hosts such as insect cells or viral expression systems such as bacteriophages like M13, T7 phage or Lambda, or viruses such as vaccinia and baculovirus expression systems.

Often, the DNA is ligated into an expression vector behind a suitable signal sequence that leads to secretion of the TNF-alpha specific protease into the extracellular space, thereby allowing direct detection of enzyme activity in the cell supernatant. Particularly suitable signal sequences for Escherichia coli, other Gram negative bacteria and other organisms known in the art include those that drive expression of the HlyA, DsbA, PhoA, PelB, OmpA, OmpT or M13 phage GIII genes. For Bacillus subtilis, particularly suitable signal sequences include those that drive expression of the AprE, NprB, Mpr, AmyA, AmyE, Blac, SacB, and for S. cerevisiae or other yeast, include the killer toxin, Bar1, Suc2, Matα, Inu1A or Ggplp signal sequence.

Alternatively, the enzyme variants are expressed intracellularly. As an alternative, after intracellular expression of the enzyme variants, or secretion into the periplasmatic space using signal sequences such as those mentioned above, a permeabilisation or lysis step is used to release the TNF-alpha specific protease into the supernatant. The disruption of the membrane barrier is effected by the use of mechanical means such as ultrasonic waves, French press, cavitation or the use of membrane-digesting enzymes such as lysozyme.

As a further alternative, the genes encoding the TNF-alpha specific protease are expressed cell-free by the use of a suitable cell-free expression system. For example, the S30 extract from Escherichia coli cells is used for this purpose as described by Lesly et al. (Methods in Molecular Biology 37, 265-278 (1995)). In cell-free systems, the gene of interest is typically transcribed with the assistance of a promoter, but ligation to form a circular expression vector is optional. Regardless of the presence of a circular vector or the final host organism, the DNA sequence of the protease expression construct is determined using techniques that are standard in the art.

### Generation of the protease: purification

As described above, the pharmaceutical proteins are expressed in a variety of expression systems and the appropriate down-stream processing and purification procedures are selected accordingly. In a preferred embodiment of the invention the protease variant is expressed in a microbial host and the protein is secreted into the periplasmic or extracellular space. Cells carrying an appropriate expressing construct for the protease variants may be preserved as cryo stocks, well known to anyone skilled in the art. Cultures for protein expression are inoculated from a cryo stock and the volume of the culture increased successively in the appropriate container. In a preferred embodiment the cells are grown in a fermenter under controlled conditions of pH, temperature, oxygen and nutrient supply. After harvesting a first step comprises the separation of cells from supernatant using one or more of several techniques, such as sedimentation, microfiltration, centrifugation, flocculation or other. In a preferred embodiment the method applied is microfiltration.

In a preferred embodiment of the invention the protein is secreted into the supernatant and a further step of purification comprises the concentration of the supernatant by ultrafiltration. Protein purification from the supernatant or concentrated supernatant is performed with one or more of several preferred chromatographic methods including but not limited to ion-exchange, hydrophobic interaction, hydroxyapatite, size fractionation by gel-filtration and affinity chromatography or any combination thereof. In a more preferred method the protein is purified by combining several ion-exchange chromatographic steps to obtain a high purity protein. An even more preferred method comprises the combination of a cation-exchange and an anion-exchange chromatography, optionally combined with further cation or anion-exchange chromatographies. An appropriate purification method yields a purity of the protein of >50%, in a more preferred method the purity is >80%, in an even more preferred method the purity is >90%, in a yet more preferred method the purity is >95% and in a most preferred method the purity is >98%.

### Measures for half-live improvement

To improve the pharmacological parameters that result in enhanced exposure to the compound and hence its pharmacological efficacy, the protease is subjected to a variety of modifications. Examples of enhanced properties include but are not restricted to an increase in circulating terminal half-life, area under the curve (AUC), solubility, decrease of immunogenicity and proteolytic degradation. These pharmacokinetic and pharmacodynamic properties of the protease of the present invention are further improved by one or more of the following strategies or any combination thereof:
a) fusion to a peptidic component, preferably being selected from, but not limited to the group consisting of binding domains, receptors, antibodies, regulatory domains, pro-sequences, serum albumin, or fragments or derivatives thereof, and/or
b) covalent conjugation to a natural or synthetic polypeptide or non-polypeptide moiety, preferably being selected from the group consisting of polyethylenglycols, carbohydrates, lipids, fatty acids, nucleic acids, metals, metal chelates, nano-particles, liposomes, dendrimers or fragments or derivatives thereof, and/or
c) introduction of consensus glycosylation sites into the protease variant that are glycosylated during the post-translational processing of the protease variant during biosynthesis and/or
d) introduction of one or more mutation, insertion, substitution or deletion that render the protease variant less sensitive to clearance mechanisms such as, but not limited to, proteolytic degradation, induction of immunogenicity or receptor mediated cellular uptake and/or
e) incorporation into formulations and/or drug delivery devices for protection and slow release.

According to strategy a) the protease is fused to a proteinacious component. In a preferred variant of this embodiment the proteinacious component is an albumin, in particular human serum albumin, fragments or derivatives or variants thereof without significantly impairing the biological properties of the protease. It causes increased terminal half-life and thus enables the protease of the present invention to maintain a given biological activity in vivo for a prolonged period. Useful albumins and fusion methods are exemplary described in US 5,876,969. Other acceptable fusion partners, among others, include the Fc portion of human immunoglobulin and human transferrin. In a preferred embodiment of the invention the protease variant is fused to human serum albumin or the Fc portion of a human IgG immunoglobulin. In a further preferred embodiment said proteinacious component is not immunogenic.

In another embodiment of this invention, fusion proteins are also generated that promote localisation of the protease to desired locations in the body such as tissues, organs or cell subtypes. The fusion partner of the protease comprises, but is not limited to, antibody fragments, for example scFv fragments with specificity for certain cellular surface antigens or other markers for a particular cell type, organ or tissue. Other fusion partners may include sequences corresponding to the binding domain within certain ligands or receptors that facilitate recognition by the protease of certain cellular surface expressed target proteins or moieties, or to circulating cytokines, growth factors, hormones, enzymes and other serum proteins. In another variant, the protease is fused to certain cytokines, growth factors, hormones, or fragments thereof, that will promote attachment to specific receptors on certain cell types to promote intake of the fusion protein to the intracellular compartment of the target cells by for example receptor mediated endocytosis. For example, the protease is fused to the whole or a fragment of the granulocyte-colony stimulating factor (G-CSF) for targeting of the G-CSF receptor on granulocytes to facilitate intracellular penetration.

According to strategy b) the protease is covalently conjugated to one or more polypeptide or non-polypeptide moieties such as polymer molecules, selected from the group described in EP-B-1062230 consisting of, but not limited to, polyethylene glycol (PEG), polyvinyl pyrrolidone, polyvinyl alcohol, polyamino acids, divinylether maleic anhydride, N-(2-Hydroxypropyl)-methacrylamide, dextran, dextran derivatives including dextran sulfate, polypropylene glycol, polyoxyethylated polyol, heparin, heparin fragments, sugar moieties such as polysaccharides, cellulose and cellulose derivatives, including methylcellulose and carboxymethyl cellulose, starch and starch derivatives, polyalkylene glycol and derivatives thereof, copolymers of polyalkylene glycols and derivatives thereof, polyvinyl ethyl ethers, and alpha, beta-Poly(2-hydroxyethyl)-DL-aspartamide, and the like, or mixtures thereof. In another preferred embodiment of strategy b the protease is covalently conjugated to polypeptides or proteins, such as but not limited to binding domains, receptors, antibodies, regulatory domains, pro-sequences, serum albumin, transferrin, the Fc portion of immunoglobulins or fragments or derivatives thereof.

In a preferred variant of this embodiment, the non-polypeptide polymer is PEG. Conjugation of PEG to proteins is a widely used technique to stabilize proteins. The PEG molecules require a chemical activation before the conjugation reaction by the introduction of a chemical activating group such as but not limited to dichlorotriazine, chlorotriazine, tresylate, succinimidyl carbonate, benzotriazole carbonate, p-nitrophenyl carbonate, trichlorophenyl carbonate, carbonylimidazole, succinimidyl succinate, propionaldehyde the generation of active esters of PEG carboxylic acids such as N-hydroxysuccinimide or carbodiimide. A specific labelling of cysteines is obtained by reaction with PEG-derivatives such as maleimide, iodoacetamide, vinylsulfone or orthopyridyl disulfide. Amine-derivatives of PEG can be covalently linked to glutamines by enzymatic reactions using enzymes such as transglutaminase (Sato, H., Advanced Drug Delivery Reviews, 54:487-504 (2002)). In the majority of cases of conjugation of PEG to peptides or proteins, electrophilic groups are introduced to the polymer which can than be coupled to the nucleophilic amino-groups of lysine residues or the N-terminal amino group on the peptide or protein. The coupling moiety is either incorporated as a part of the PEG-protein conjugate or lost fully or partially during the coupling step. Different pegylation methods and chemistries and their effect on biopharmaceuticals are well known by a person skilled in the art (Roberts, M.J. et al., Advanced Drug Delivery Reviews, 54:459-476 (2002); Harris, J.M. and Chess, R.B., Nature Reviews, 2:214-221 (2003)). The PEG moiety can be of different structure. In a preferred embodiment of the invention the terminal hydroxyl group is methylated (mPEG). The structure of the PEG molecules is either linear, bifurcated or branched. In a branched PEG two or more PEG molecules are connected to a moiety providing the branching point and the connectivity to a reactive chemical group for attachment to the target protein. The average molecular weight of the reactant PEG is preferably between about 5,000 and about 60,000 Daltons, more preferably between about 10,000 and about 50,000 Daltons, and most preferably between about 20,000 and about 40,000 Daltons. Particularly preferred are PEGs having nominal average sizes of about 20,000 and about 40,000 Daltons. The method of attachment is not critical, but preferably does not alter, or only minimally alters, the activity of the biologically active molecule. Preferably the increase in terminal half-life outweighs any decrease in biological activity. In a most preferred embodiment of this aspect of the invention the PEG-moiety is covalently linked to lysine residues using a N-hydroxysuccinimide-activated, methylated PEG-derivative of a nominal size between 5kD and 60kD.

In another preferred variant of this embodiment the non-polypeptide moiety is a glycoside. Covalent *in-vitro* coupling of glycosides to amino acid residues of the protease may be used to modify or increase the number or profile of carbohydrate substituents. Depending on the coupling mode used, the one or more sugar moieties may be attached to a) lysine, arginine and histidine, b) free carboxyl groups, c) free sulfhydryl groups such as those of cysteine, d) free hydroxyl groups such as those of serine, threonine, tyrosine or hydroxyproline, e) aromatic residues such as those of phenylalanine or tryptophan or f) the amide group of glutamine. These amino acid residues constitute examples of attachment groups for a sugar moiety, which may be introduced and/or removed in the protease of the present invention. Suitable methods of in vitro coupling are described, for example, in WO8705330 A1. The in vitro coupling of sugar moieties or PEG to protein-and peptide-bound Gin-residues can also be carried out by transglutaminases, e. g. as described in EP-A-725145.

According to strategy c) of this embodiment the non-polypeptide moiety is a carbohydrate molecule attached by *in-vivo* or *in-vitro* post-translational glycosylation, such as N- or O-glycosylation. The *in-vivo* glycosylation consensus sequence is asparagine-x-serine or asparagine-x-threonine throughout the eukaryotic kingdom, where x stands for any amino acid except proline. The improvement of the pharmacokinetic properties of several proteins through the introduction of consensus glycosylation sequences has been reported (Elliot et al., Nature Biotech, 21:414-421 (2003)). In order to obtain a glycosylation structure similar or identical to human glycosylation a yeast host has been engineered that expresses human enzymes involved in the generation of glycosyl structures. This strain produces glycosylated protein that is virtually identical to human protein (Hamilton et al., Science, 301:1244-1246 (2003)). In a preferred aspect of this embodiment of the invention the 9 base pairs coding for the consensus glycosylation sites are introduced in frame into the coding region of the gene for the protein variant where the 9 base pairs can replace either none, three, six or nine base pairs at the position of insertion. This leads to the insertion of three amino acids N-X-S/T or the full or partial replacement of amino acids of the variant protease. In a preferred embodiment of the invention, the insertion site can be random or based on rational design. The engineered gene of the protease variant containing additional glycosylation sites is introduced in an appropriate expression vector in an expression host, selected from filamentous fungi or yeast, insect or animal cells, from transgenic plant cells or any other suitable eukaryotic expression host. Furthermore, the glycosylation may be achieved in the human body when using a nucleotide sequence encoding the polypeptide of the invention in gene therapy. In one embodiment the host cell is a mammalian cell, such as a Chinese Hamster Ovary (CHO) cell, a BHK cell or a HEK cell, e. g. a HEK293 cell, or an insect cell, such as an SF9 cell, or a yeast cell, e. g. Saccharomyces cerevisiae, Pichia pastoris, Kluveromyces lactis or any other suitable glycosylating host. Optionally, sugar moieties attached to the protease by in vivo glycosylation are further modified by use of glycosyltransferases or other processing enzymes.

According to strategy d) of this embodiment mutations, insertions or deletions are introduced into the coding gene of the protease variant leading to one or more amino acid changes in the protein. In a preferred embodiment said mutant protein has an increased terminal half-life compared to the non-mutated variant. In a preferred aspect of this embodiment said mutation renders the variant less sensitive to proteolytic degradation by proteases that the protease variant of the invention comes into contact with in the human body. The introduction of the mutation is either based on rational design after experimentally identifying cleavage sites or by screening a library of protease variants under suitable conditions allowing the selection of protease variants less sensitive to degradation. The principle has been demonstrated by Gillies and co-workers (Gillies et al., Clinical Cancer Research, 8:210-226 (2002)). In a further aspect of this embodiment introduction of changes in the amino acid sequence of the protease leads to the reduction or elimination of undesired interactions of the protease, or parts thereof, with one or more components of the surroundings of the protease, such as but not limited to, serpins, cell-surface receptors or molecules, components of the extracellular matrix, soluble components of the serum or any other body fluid. Again, the amino acid exchange can be introduced based on rational design or mutated protease variants can be selected in a screening process comprising a library of protease variants and an appropriate screening process, allowing the identification of protease variants characterized by a reduction in an undesired interaction with certain components of the surroundings.

According to strategy e) the protease may be formulated in such a way that the protease is protected from premature clearance and/or degradation by external factors and is released into circulation in a controlled manner, in order to optimize its pharmacokinetic profile such as the systemic exposure of the drug, the area under the curve or terminal half-life of the protease. These formulations allow for increased dosing levels of the drug that can be administered, reduce concentration differences between peak and trough levels and allow a more efficient use of the therapeutic window. Therapeutic formulations are typically sterile and exhibit long-term stability under the conditions of manufacture and storage. Examples such as the entrapment in a matrix or the encapsulation in a semipermeable membrane represent some of the most widely used stabilisation techniques (Ingemann M. et al., Pharmaceutical Formulation Development of Peptides and Proteins by Frokjaer, S. and Hovgaard, L., 1st edition, chapter 10 (pages: 189-205) (2000)). According to this strategy the entrapment or encapsulation may be done by a polymeric drug delivery system, such as hydrogel or nanocapsule / microsphere, or lipid drug delivery system such as liposomes and microemulsions. A further option is the use of micropumps for the controlled release of the drug in a suitable formulation.

### Indications and dosing

As detailed above, hTNF-alpha has been implicated in the ethyology and/or progression of multiple diseases. The protease variants of the invention can be used for preparing medicinal drugs for the treatment of diseases and various disorders associated with TNF-alpha. The invention provides methods to reduce the hTNF-alpha activity in individuals suffering from said diseases, comprising the administration of protease variants to the subject in a suitable form and dosage which results in a reduction of hTNF-alpha activity to a degree that ameliorates or reduces signs and symptoms of the disease or disorder. The subject is an animal or more preferably a mammal, which expresses TNF-alpha, that serves as a target for the protease variant of the invention. Most preferable the subject is a human. Diseases that can be treated with the protease variants include, but are not limited to, the following:
*Autoimmune diseases:* rheumatoid arthritis, juvenile rheumatoid arthritis, psoriasis, psoriatic arthritis, ankylosing sponndylitis, sclerotic arthritis, Behcet's disease, adult-onset Still's disease, autoimmune uveitis, multiple sclerosis, insulin resistance in diabetes, Sjogren's Syndrome, systemic lupus erythematosus, Systemic inflammatory response syndrome (SIRS) which leads to distant organ damage and multiple organ dysfunction syndrome (MODS),
*Intestinal diseases:* inflammatory bowel diseases, Crohn's disease, ulcerative colitis;
*Infectious diseases:* sepsis, viral diseases and infections, encephalitis, bacterial meningitis, cerebral malaria, AIDS and AIDS-related complex, cytomegalovirus infection secondary to transplantation;
*Pulmonary disorders:* chronic obstructive pulmonary disease, asthma, pulmonary sarcoidosis, pulmonary fibrosis, silicosis, shock lung;
*Malignancy:* neoplastic diseases associated with abnormal immune function, classical Hodgkin's Lymphoma (cHL), cachexia secondary to malignancy;
*Cardio-vascular disorders:* arterial sclerosis, hypertension, vasculatitis, disorders of haematopoietic cells, heart failure, stroke, vasodilation, intravascular coagulation
*Transplantation:* graft-versus-host disease;
*Other disorders:* osteoporosis, scleroderma, polymyositis, dermatomyositis, Grave's disease, Hashimoto's thyroiditis, eosinophilia, neurodegenerative disease, closed head injury and multiple organ failure.

Preferably, the invention is indicated for the treatment of rheumatoid arthritis, juvenile rheumatoid arthritis, Crohn's disease, psoriasis, psoriatic arthritis, ankylosing sponndylitis, systemic lupus erythematosus and ulcerative colitis, where blocking anti-TNF-alpha immunoglobulin derived biotherapeutics have validated the target in a clinical setting. Second most favoured indications are chronic obstructive pulmonary disease, sepsis, Behcet's disease, adult-onset Still's disease, polymyositis, dermatomyositis, osteoporosis, uveitis and vasculatitis, as supported by preclinical evidence for a role for TNF-alpha in the modulation of the underlying molecular mechanisms of these inflammatory diseases.

For all of the above indications the preferred dosing regimen for the protease is administration as a monotherapy. The second most favoured regimen comprises the co-administration of non-biological therapies that are standard of care in the treatment of the above diseases and disorders. These include but are not restricted to disease modifying anti-rheumatic drugs, corticosteroids, non-steroidal anti-inflammatory drugs and analgesics. Examples of these compounds include methotrexate, cyclosporine A, azathioprine, and mesalazine. The most preferred compound is methotrexate. The second most preferred compound is cyclosporine A. In a third most preferred dosing regimen, the protease of the invention is co-administered with protein-based, anti-TNF-alpha specific or otherwise, therapeutics to provide additive or synergistic efficacy. Examples include among others, Fc-fusion proteins such as Etanercept, monoclonal antibodies such as infliximab and adalimumab in the treatment of for example RA, or efalizumab and alefacept in the treatment of for example psoriasis. Furthermore, the invention is compatible with co-administration with other non-biological compounds or agents that are utilized in the clinic to ameliorate the symptoms of the disease or reduce the scope of drug induced adverse events.

The diseases are treated by administering to a patient a therapeutically effective amount of a composition comprising the TNF-alpha specific protease. The specific amount and frequency of the active compound administered will depend on the indication, supporting data from clinical trial studies in the given indication, the subject being treated, the subject's weight, the formulation and manner of administration, and the judgment of the prescribing physician. Information concerning dosages of various pharmacological agents is found in standard pharmaceutical reference books, e.g.

Remington, The Science And Practice Of Pharmacy, 21st edition (2005). The definition of the amount and frequency of administration of a composition of the invention that will be effective in the treatment of a particular disorder or condition disclosed herein will depend on the nature of the disorder or condition, and is determined by standard clinical techniques. In addition, in vitro or in vivo assays are optionally employed to help identify optimal dosage ranges. The precise dose of the compositions to be employed will also depend on the route of administration, and the seriousness of the disease or disorder, and should be decided according to the judgment of clinical trial results, the practitioner and each patient's circumstances.

The effective dose of the TNF-alpha specific protease may be determined in a number of ways, including dosages calculated to alleviate symptoms associated with a specific disease state in a patient. Alternatively, dosages are calculated to comprise an effective amount of the TNF-alpha specific protease to induce a detectable change in TNF-alpha levels in the blood, synovial fluid or any other body fluid or compartment. Such detectable changes in blood cytokine concentrations include a decrease in hTNF-alpha levels of between about 1% and 99%, or between about 5% and about 80%. In other instances, the protease variant is given at a dose sufficient to detect activity of the hTNF-alpha specific protease in the patient through the measurement of defined secondary pharmacodynamic endpoints. Formulations are also calculated using a unit measurement of activity of hTNF-alpha specific protease. The measurements by weight or activity can be calculated using known standards.

However, suitable dosage ranges for intravenous, epidural or intracerebral administration are preferably between 0.1 to 50 mg per kilogramme body weight. Suitable dosage ranges for intranasal and pulmonary administration are generally about 0.5 to 100 mg per kilogramme body weight. Suppositories and orally administered tablets and pills generally contain 0.5 to 100 mg per kilogramme of the protease of the invention per kilogramme body weight. Recommended dosages for intradermal, intramuscular, intraperitoneal, subcutaneous, sublingual, intravaginal or transdermal administration are in the range of 1 to 100 mg per kilogramme of body weight. Administrations by inhalation are in the range of 1 to 100 mg per kilogramme of body weight. Suitable doses of the compounds of the invention for topical administration are in the range 0.25 to 25 mg per kilogramme body weight, depending on the surface area to which the compound is administered.

The composition comprising the protease of the invention may, though not necessarily, be administered daily, in an effective amount to ameliorate a disease symptom. Preferably, it is administered once every other day, or more preferably once a week, or even more preferably every other week, or even more preferably at intervals longer than two weeks.

### Application and general formulation of the protease

The hTNF-alpha specific protease is administered to achieve efficacious concentration levels in target tissues that maximize its disease modulating effects. It may be administered by any convenient route, for example, intradermal, intramuscular, intraperitoneal, intravenous infusion or bolus injection, subcutaneous, intranasal, epidural, oral, sublingual, intracerebral, intravaginal, transdermal, rectally, by inhalation, or topically, by absorption through epithelial or mucocutaneous linings (e.g., oral mucosa, rectal and intestinal mucosa, etc.). It is administered in a formulation suitable for the respective route of administration and is optionally co-administered together with another biologically or chemically active agent. Preferably the formulation has the broadest applicability. For example protease in a given formulation can interchangeably be administered subcutaneously, intramuscularly or intravenously.

In a further embodiment of this aspect, it may be desirable to administer one or more compositions of the invention locally to the area in need of treatment. This is achieved, for example, and not by way of limitation, by topical application (e. g., as a cream), by local infusion during surgery, by injection, by means of a catheter, by means of a suppository, by means of inhalation or by means of an implant. In one embodiment, administration can be by direct injection at the site (or former site) of the tissue.

The administration and compositions of the invention are preferably assayed in vitro and in vivo in relevant animal disease models, for the desired therapeutic or prophylactic activity, prior to use in humans. For example, in vitro assays are used to determine whether administration of a specific composition of the invention or a combination of compositions of the invention is preferred for treating or ameliorating a disease or disorder as described herein. The compositions of the invention are also demonstrated to be effective and safe using animal model.

Therapeutic compositions typically must be sterile and stable under the conditions of manufacture, storage and application. Administration is systemic or local. Various delivery systems are known, for example, encapsulation in liposomes, microparticles, microcapsules, capsules, etc. as described above, and used to administer a composition of the invention. In certain embodiments, more than one composition of the invention is administered to a patient. The preferred mode of administration is chosen according to the results of clinical trial data and left to the discretion of the practitioner, and will depend in-part upon the site and severity of the medical condition. In most instances, administration will result in the release of the composition of the invention for maximum efficacy.

In the case of parenteral administration, the compositions may be encapsulated in a liposome envelope that is coupled to a (poly)peptide (e.g. an antibody) or chemical moiety directed against specific proteins and other cell surface structures so as to provide target tissue selectivity. The specific nature of the formulation is also determined by the desired route of administration, e.g., topical, parenteral, oral, rectal, surgical implantation or by other means of local (intraprostatic) delivery. The dosage is determined for each route of administration to maximize pharmacokinetic profile and efficacy. The amount of TNF-alpha specific protease in the composition ranges from about 0.01 to 99% by weight of the composition.

Thus, a further aspect of the present invention is directed to pharmaceutical compositions comprising the TNF-alpha specific protease. Suitable forms of the compositions are solutions, suspensions, emulsion, tablets, pills, pellets, capsules, capsules containing liquids, powders, sustained-release formulations, suppositories, emulsions, aerosols, sprays, suspensions, or any other form suitable for use. Other examples of suitable pharmaceutical vehicles are described in Remington, The Science And Practice Of Pharmacy, 21st edition (2005). The compositions are prepared by mixing the TNF-alpha specific protease with any pharmaceutically acceptable component, such as, for example, a carrier, a medicinal agent, an adjuvant, a diluent, and the like, as well as combinations thereof. Suitable pharmaceutical carriers, medicinal agents, adjuvants, and diluents are also described in Remington: The Science And Practice Of Pharmacy, 21st edition (2005). When administered to a patient, the composition is preferably sterile. Therefore, water is a preferred vehicle when the compound of the invention is administered intravenously. Other applicable liquid vehicles are preferably derived from natural sources, e.g. of petroleum, animal or vegetable origin. These include among others saline, urea, peanut oil, soybean oil, sesame oil, gum acacia, gelatin, keratin and starch paste. Chemically derived liquids such as mineral oils, talc, colloidal silica, aqueous dextrose and glycerol solutions among others are also suitable. Suitable pharmaceutical vehicles also include excipients such as, but not limited to, starch, glucose, lactose, sucrose, malt, rice, flour, chalk, silica gel, sodium stearate, glycerol monostearate, talc, sodium chloride, dried skim milk, glycerol, propylene, glycol, ethanol, mannitol, polysorbate 20, polysorbate 80, trehalose and the like. In addition, auxiliary, stabilizing, thickening, lubricating and colouring agents may be used. The composition may comprise stabilisers such as but not limited to sucrose, glucose, polyols, trehalose, mannitol, polysorbate 20, polysorbate 80, sodium chloride, buffer salts, ion exchange polymers and others. Further components of the composition are wetting or emulsifying agents, surfactants or pH buffering agents.

### Intravenous administration

In another embodiment, the compositions of the invention are formulated in accordance with routine procedures as a pharmaceutical composition adapted for intravenous administration to human beings. Typically, compositions of the invention for intravenous administration are solutions in sterile isotonic aqueous buffer. Where necessary, the compositions also include a solubilising agent. Compositions for intravenous administration optionally include a local anaesthetic such as lignocaine to ease pain at the site of the injection. Preferably, the ingredients are supplied as a dry lyophilized powder or water free concentrate in a hermetically sealed container such as an ampoule or sachette, each containing the exact quantity of active agent for a single application. Typically the ingredients are reconstituted to the final injectable concentrations by the addition of sterile water or saline provided separately and mixed just prior to administration. Where the composition of the invention is to be administered by intravenous infusion, it is dispensed by a bolus injection or by continuous injection, for example, with an infusion bottle containing sterile pharmaceutical grade water or saline.

### Slow release formulations for parenteral administration

In order to increase systemic exposure of the drug, the area under the curve or terminal half-life of the protease, a particular preferred embodiment comprises slow-release formulations. They reduce concentration differences between peak and trough levels and allow a more efficient use of the therapeutic window. Examples for slow-release formulations are the entrapment in a matrix or the encapsulation in a semipermeable membrane, the most widely used stabilization techniques (Ingemann M. et al., Pharmaceutical Formulation Development of Peptides and Proteins by Frokjaer, S. and Hovgaard, L., 1st edition chapter 10 (pages: 189-205) (2000)). According to this strategy the entrapment or encapsulation is done by a polymeric drug delivery system, such as hydrogel or nanocapsule / microsphere, or lipid drug delivery system such as liposomes and microemulsions. A further option is the use of micropumps for the controlled release of the drug in a suitable formulation.

In a first variant of this embodiment hydrogels are crosslinked via hydrophilic polymers of natural or synthetic origin to entrap the protease drug. Hydrogels have the ability to swell in an aqueous environment without dissolving. By hydrogel entrapment monomers form a crosslinked polymeric network around the material to be entrapped. The surrounding polymer matrix permits entrapment of large quantities of protein drugs. The polymerization reaction is carried out in bulk, solution or suspension by free radical polymerization initiated by the generation of free radicals by thermal, ionization or redox means. This is performed by mixing of monomers and cross-linking agent in an organic solvent or buffered solution followed by the addition of a catalyst system, which initiates the polymerization process. The protease to be entrapped can be introduced into the hydrogel matrix during or after synthesis. Entrapment after synthesis is done by soaking of pre-washed hydrogels in a concentrated drug solution. In a preferred variant the protease is introduced after cross-linking. The polymerized protease complex may be produced in a wide variety of geometric shapes including films, membranes, rods and particles. Examples of synthetic and natural polymers which are used for hydrogels are acrylics, vinyl alcohols, ethylene oxides, cellulose ethers and starch, albumin or dextran. In addition hydrogels with enzyme digestible crosslinkers or polymer backbones are used as biodegradable drug delivery systems. A further example for an encapsulating material could comprise peptidic components that are slowly degraded by the proteolytic action of the protease variants of the invention. These peptidic components could for example contain the target sequence or variants thereof of the specific protease. The peptidic components are connected in such a way as to effectively entrap the proteases and cleavage of the peptidic component leads to a release of the protease.

In a second variant of this embodiment the protease of the present invention is encapsulated by nanocapsules, nanospheres, microcapsules or microspheres which mainly differ by their size of below and above 1 µm and the process used for preparation. Nanocapsules and microcapsules are vesicular systems in which the drug is confined to a cavity surrounded by a polar membrane, whereas nanospheres or microspheres are matrix systems in which the protease is dispersed throughout the particle. There are two common preparation methods in preparation of nanocapsules and microcapsules according to whether the formation requires an *in situ* polymerization reaction or whether it is achieved directly from a preformed polymer or a natural macromolecule via precipitation of synthetic polymers or by denaturation of natural macromolecules. In contrast, common processes to prepare nanospheres and microspheres include spray-drying, solvent-evaporation and phase-separation techniques. Microspheres comprising the protease of the invention are commonly made out of polystyrene, poly-methylmethacrylate, poly-hydroxybutyrate, poly-D,L-lactic acid, poly-L-lactic acid, poly-D,L-lactide-coglycolide, ethyl cellulose, cellulose acetate, hydrogen phthalate or cellulose triacetate

In a third variant of this embodiment the protease is included into liposomes which are considered for parenteral administration (Torchilin, Nat. Rev. Drug Discov., 4:145-160 (2005)). Liposomes are vesicles in which an aqueous core is enclosed by phospholipid bilayers. They are broadly classified by size and lamellarity as small unilamellar vesicles (SUVs: size 25-50 nm), large unilamellar vesicles (LUVs: 100 nm) and multilamellar vesicles (MLVs: 50-10000 nm). Liposomes are spontaneously formed when phospholipids are dispersed in excess water, arranging themselves in bilayers with the enclosure of an aqueous core. For SUVs and MLVs is a lipid hydration step involved, whereas LUVs are made by solvent dispersion methods. In a preferred embodiment the surface of the liposomes is modified to increase circulating half-life in the blood. Suitable surface modifications comprise but are not limited to covalent coupling of polyethylene glycol (PEG), poly[N-(2-hydroxypropyl)methacrylamide], poly-N-vinylpyrrolidones, L-amino-acid-based biodegradable polymer-lipid conjugates, polyvinyl alcohols, mucins or sugar residues such as mannosyl. In a further preferred embodiment the liposome surface is modified to include a targeting moiety or moieties. Examples are antibodies, receptors, receptor ligands, binding domains or fragments thereof. In another aspect of the embodiment the liposomes are pH-sensitive, i.e. they are stable at the blood pH but destabilized at either lower or higher pH compared to the pH in the blood. pH-sensitive liposomes can be generated by methods known in the art, for example by use of anionic phospahtidylethanolamine or terminally alkylated co-polymer of N-isopropylacrylamide and methyacrylic acid. In a preferred embodiment the protease is included in liposomes with any combination of surface modification, targeting moiety and pH-sensitivity. In a most preferred embodiment the liposomes are pegylated and/or carry a targeting moiety on the surface that allows direction to the desired locus and/or are destabilised at low pH. Liposomes are also a preferred formulation for oral or pulmonary delivery.

In a fourth variant of this embodiment microemulsions are used to formulate a drug delivery system for the protease. Emulsions are two immiscible phases dispersed in one another. Microemulsions are more complex quarternary or ternary systems, containing a surfactant/co-surfactant blend added to a two-phase hydrophilic / lipophilic mixture. Microemulsions are spontaneously formed at room temperature, appear transparent, and consist of micro-droplets of a size less than 100 nm. The pharmaceutical compositions may be in the form of a sterile injectable preparation, for example, as a sterile injectable aqueous or oleaginous suspension. This suspension may be formulated according to the known art using those suitable dispersing or wetting agents and suspending agents. The sterile injectable preparations may also be a sterile injectable solution or suspension in a non-toxic parenterally-acceptable diluent or solvate, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

In a fifth variant of this embodiment the compositions of the invention are delivered in a controlled release system, such as a pump or micropump. These include devices consisting of an osmotic core with the drug surrounded by a semipermeable membrane drilled with a delivery orifice in which the drug is delivered passively or by the use of different channeling agents in the coating to control release. In another approach, the protease is delivered for extended periods using diffusion-controlled implanted tubes. These devices are designed to release drugs at various dosages and for both intermittent and continuous delivery. Infusion times are designed to operate for varying periods of time from days to several months. In yet another approach, microscale pumps are used in which the protease is delivered by ingestion, injected into tissue, inhaled or even released into circulation.

In yet another embodiment, any of the controlled-release systems described above is placed in proximity of the target area to be treated, thus requiring only a fraction of the systemic dose. All compositions used for controlled-release formulations will contain a therapeutically effective amount of the hTNF-alpha specific protease, optionally with an additional therapeutic, preferably in purified form, together with a suitable amount of a pharmaceutically acceptable vehicle so as to provide the form for proper administration to the patient.

### Topical / transdermal administration

In another embodiment, the composition is prepared in a form suitable for administration directly or indirectly to surface areas of the body for direct application to affected areas. This formulation includes, but is not limited to, anti-drying agents (e. g. pantethine), penetration enhancers (e. g dimethyl isosorbide), accelerants (e.g., isopropylmyristate) or other common known additives used for topical applications (e. g. glycerin, propylene glycol, polyethylene glycols, ethyl alcohol, liposomes, lipids, oils, creams, or emollients).

Most drugs are not able to cross the stratum corneum. However, enhanced penetration is achieved using a class of compounds known collectively as penetration enhancers. Alcohols, sulphoxides, fatty acids, esters, azone, pyrrolidones, urea and polyols are just some of the members of this class of compounds (Kalbitz et al., Modulation of drug penetration in the skin, Pharmazie 1996; 51(9):619-637). The objectives of these penetration enhancers are to change the solubility and diffusivity of the drug in the stratum corneum, thus some modulate their effects through the lipid pathway while others modify diffusion via the polar pathway.

Various concentrations of the enhancer glycerine have been shown to enhance the penetration. The use of terpene-based penetration enhancers with aqueous propylene glycol have also shown the capacity to enhance topical delivery. Dimethyl isosorbide (DMI) is another penetration enhancer that has shown promise for pharmaceutical formulations. DMI is a water-miscible liquid with a relatively low viscosity that undergoes complexation with water and polyene glycol but not polyethylene glycol. It is the ability for DMI to complex with water that provides the vehicle with the capacity to enhance the penetration of various steroids. Maximum effects were seen at a DMI: water ratio of 1: 2. Evidence in the literature suggests that the effect of pH on DMI is an important consideration when using DMI in various formulations.

The present invention also provides formulations of TNF-alpha specific protease for transdermal delivery. Transdermal systems deliver therapeutic formulations through the skin into the bloodstream, making them easy to administer. Passive and active transdermal delivery systems are used to deliver medicines in even concentrations in a way that is painless and results in few adverse side effects. The fusion proteins could be delivered transdermally using a skin patch and other means such as microneedles, i.e. mechanically puncturing the skin in order to increase the permeability of the skin to a drug.

### Pulmonary administration

Moreover, the present invention includes pulmonary delivery of the TNF-alpha specific protease formulations. Pulmonary delivery is particularly promising for the delivery of macromolecules, which are difficult to deliver by other routes of administration. Such pulmonary delivery can be effective both for systemic delivery and for localized delivery to treat diseases of the lungs, since drugs delivered to the lung are readily absorbed through the alveolar region directly into the blood circulation.

The invention further provides compositions suitable for forming a drug dispersion for oral inhalation (pulmonary delivery) to treat various conditions or diseases. The TNF-alpha specific protease formulation could be delivered by different approaches such as liquid nebulizers, aerosol based metered dose inhalers (MDI's), and dry powder dispersion devices. In formulating compositions for pulmonary delivery, pharmaceutically acceptable carriers including surface active agents or surfactants and bulk carriers are commonly added to provide stability, dispersibility, consistency, and/or bulking characteristics to enhance uniform pulmonary delivery of the composition to the subject.

Surface active agents or surfactants promote absorption of polypeptide through mucosal membrane or lining. Useful surface active agents or surfactants include fatty acids and salts thereof, bile salts, phospholipids, or an alkyl saccharide. Examples of fatty acids and salts thereof include sodium, potassium and lysine salts of caprylate (C8), caprate (CI0), laurate (C12) and myristate (CI4). Examples of bile salts include cholic acid, chenodeoxycholic acid, glycocholic acid, taurocholic acid, glycochenodeoxycholic acid, taurochenodeoxycholic acid, deoxycholic acid, glycodeoxycholic acid, taurodeoxycholic acid, lithocholic acid, and ursodeoxycholic acid.

Examples of phospholipids include single-chain phospholipids, such as lysophosphatidylcholine, lysophosphatidylglycerol, lysophosphatidylethanolamine, lysophosphatidylinositol and lysophosphatidylserine; or double-chain phospholipids, such as diacylphosphatidylcholines, diacylphosphatidylglycerols, diacylphosphatidylethanolamines, diacylphosphatidylinositols and diacylphosphatidylserines. Examples of alkyl saccharides include alkyl glucosides or alkyl maltosides, such as decyl glucoside and dodecyl maltoside.

Pharmaceutical excipients that are useful as carriers include stabilizers such as human serum albumin (HSA) or recombinant human albumin; bulking agents such as carbohydrates, amino acids and polypeptides; pH adjusters or buffers; salts such as sodium chloride; and the like. These carriers may be in a crystalline or amorphous form or may be a mixture of the two.

Examples of carbohydrates for use as bulking agents include monosaccharides such as galactose, D-mannose, sorbose, and the like; disaccharides, such as lactose, trehalose, and the like; cyclodextrins, such as 2-hydroxypropyl-betacyclodextrin; and polysaccharides, such as raffinose, maltodextrins, dextran, and the like; alditols, such as mannitol, xylitol, and the like. Examples of polypeptides for use as bulking agents include aspartame. Amino acids include alanine and glycine, with glycine being preferred.

Additives, which are minor components of the composition, are included for conformational stability during spray drying and for improving dispersibility of the powder. These additives include hydrophobic amino acids such as tryptophan, tyrosine, leucine, phenylalanine, and the like.

Suitable pH adjusters or buffers include organic salts prepared from organic acids and bases, such as sodium citrate, sodium ascorbate, and the like; sodium citrate is preferred.

The TNF-alpha specific protease compositions for pulmonary delivery are packaged as unit doses where a therapeutically effective amount of the composition is present in a unit dose receptacle, such as a blister pack, gelatin capsule, or the like. The manufacture of blister packs or gelatin capsules is typically carried out by methods that are generally well known in the packaging art.

One approach for the pulmonary delivery of dry powder drugs utilizes a handheld device with a hand pump for providing a source of pressurized gas. The pressurized gas is abruptly released through a powder dispersion device, such as a venturi nozzle, and the dispersed powder made available for patient inhalation.

The aerosolization of protein therapeutic agents is disclosed in EP-B-0 289 336. Therapeutic aerosol formulations are disclosed in WO 90/09781.

The present invention provides formulating TNF-alpha specific protease for oral inhalation. The formulation comprises TNF-alpha specific protease and suitable pharmaceutical excipients for pulmonary delivery. The present invention also provides administering the fusion protein composition via oral inhalation to subjects in need thereof.

### Rectal administration

Compositions for rectal administration are prepared with any of the usual pharmaceutical excipients, including for example, binders, lubricants and disintegrating agents. The composition may also include cell penetration enhancers, such as aliphatic sulphoxides. In a preferred embodiment, the composition is in the form of a suppository.

### Oral administration

Proteins and peptides are prone to chemical and conformational instability and are often degraded by the acidic conditions in the stomach, as well as by enzymes in the stomach and gastrointestinal tract. However, to allow oral delivery of proteins and peptides, certain technologies for oral delivery have been developed, such as encapsulation in nanoparticles composed of polymers with a hydrophobic backbone and hydrophilic branches as drug carriers, encapsulation in microparticles, insertion into liposomes in emulsions, and conjugation to other molecules. In a particular embodiment of the invention, the protease variant is formulated for oral delivery exploiting one or several of the technologies developed for oral delivery of proteins.

Compositions of the invention for oral delivery are in the form of tablets, lozenges, aqueous or oily suspensions, granules, powders, emulsions, capsules, syrups, or elixirs. Compounds and compositions of the invention for oral delivery can also be formulated in foods and food mixes. Orally administered compositions contain one or more optionally agents, for example, sweetening agents such as fructose, aspartame or saccharin; flavouring agents such as peppermint, oil of wintergreen, or cherry; colouring agents; and preserving agents, to provide a pharmaceutically palatable preparation. Moreover, where in tablet or pill form, the compositions are coated to delay disintegration and absorption in the gastrointestinal tract thereby providing a sustained action over an extended period of time. Selectively permeable membranes surrounding an osmotically active driving compound are also suitable for orally administered compositions of the invention. In these later platforms, fluid from the environment surrounding the capsule is imbedded by the driving compound, which swells to displace the agent or agent composition through an aperture.

These delivery platforms can provide an essentially zero order delivery profile as opposed to the spiked profiles of immediate release formulations. A time delay material such as glycerol monostearate or glycerol stearate may also be used. Oral compositions can include standard vehicles such as mannitol, lactose, starch, magnesium stearate, sodium saccharine, cellulose, magnesium carbonate, etc. Such vehicles are preferably of pharmaceutical grade.

The TNF-alpha specific protease that is used to treat certain classes of a diseases or medical conditions are particularly amenable for oral formulation and delivery, e.g. inflammatory diseases of the gastro-intestinal tract such as Crohn's disease and ulcerative colitis. In many chronic inflammatory diseases as described above, oral formulations of the TNF-alpha specific protease and methods of administration are particularly useful because they allow long-term patient care and therapy via home oral administration without reliance on injectable treatment or drug protocols.

Oral formulations and delivery methods comprising the TNF-alpha specific protease take advantage of, in part, various receptor mediated transcytosis across the gastrointestinal (GI) epithelium. For example, the transferrin receptor is found at a very high density in the human GI epithelium, transferrin is highly resistant to tryptic and chymotryptic digestion and transferrin chemical conjugates have been used to successfully deliver proteins and peptides across the GI epithelium (Xia et al., J. Pharmacol. Experiment. Therap., 295: 594-600 (2000); Xia et al., Pharmaceutical Res., 18 (2): 191-195 (2001); and Shah et al., J. Pharmaceutical Sci., 85 (12): 1306-1311 (1996)). In a further embodiment the protease is fused to the constant region of an immunoglobulin (Fc) which can interact with the Fc-receptor in the gut epithelium and be transported to the blood stream.

Oral formulations of TNF-alpha specific proteases are prepared so that they are suitable for transport to the GI epithelium and protection of the fusion protein component and other active components in the stomach. Such formulations include carrier and dispersant components and be in any suitable form, including syrups, elixirs, tablets, including chewable tablets, hard or soft capsules, troches, lozenges, aqueous or oily suspensions, emulsions, cachets or pellet granulates and dispersible powders. Preferably, formulations comprising the TNF-alpha specific protease are employed in solid dosage forms suitable for simple, and preferably oral, administration of precise dosages. Solid dosage forms for oral administration are preferably tablets, capsules, or the like.

For oral administration in the form of a tablet or capsule, care is taken to ensure that the composition enables sufficient active ingredient to be absorbed by the host to produce an effective response. Thus, for example, the amount of the TNF-alpha specific protease is increased over that theoretically required or other known measures such as coating or encapsulation taken to protect the polypeptides from enzymatic action in the stomach.

In a particularly preferred embodiment, oral pharmaceutical compositions comprising the TNF-alpha specific protease are formulated in buffered liquid form which is then encapsulated into soft or hard-coated gelatin capsules which are then coated with an appropriate enteric coating. For the oral pharmaceutical compositions of the invention, the location of release is anywhere in the GI system, including the small intestine (the duodenum, the jejunum, or the ileum), or the large intestine.

Examples of nanoparticles include mucoadhesive nanoparticles coated with chitosan and Carbopol (Takeuchi et al., Adv. Drug Deliv. Rev., 47(1):39-54, (2001)) and nanoparticles containing charged combination polyesters, poly (2-sulfobutyl-vinyl alcohol) and poly (D, L- lactic-co-glycolic acid) (Jung et al., Eur. J. Pharm. Biopharm., 50(1):147-160 (2000)). Nanoparticles containing surface polymers with poly-N-isopropylacrylamide regions and cationic poly-vinylamine groups showed improved absorption of salmon calcitonin when administered orally to rats. Drug delivery particles composed of alginate and pectin, strengthened with polylysine, are relatively acid and base resistant and can be used as a carrier for drugs. These particles combine the advantages of bioadhesion, enhanced absorption and sustained release (Liu et al., J. Pharm. Pharmacol., 51(2): 141-149 (1999)).

Another formulation example is in poly (vinyl alcohol) gel spheres, such as aprotinin or bacitracin. The glucose-lowering properties of these gel spheres have been demonstrated in rats, where insulin is released largely in the lower intestine (Kimura et al., Biol. Pharm. Bull. 19 (6): 897-900,1996). Oral delivery has also been studied using nanoparticles made of poly (alkyl cyanoacrylate) that were dispersed with a surfactant in an oily phase (Damge et al., J. Pharm. Sci., 86 (12): 1403-1409 (1997)) and using calcium alginate beads coated with chitosan (Onal et al., Artif. Cells Blood Substit. Immobil. Biotechnol. 30(3):229-237 (2002)).

Compositions comprising TNF-alpha specific proteases intended for oral use are prepared according to any method known for the manufacture of pharmaceutical compositions and such compositions contain one or more agents including, but not limited to, sweetening agents in order to provide a pharmaceutically elegant and palatable preparation. For example, to prepare orally deliverable tablets, a TNF-alpha specific protease is mixed with at least one pharmaceutical excipient, and the solid formulation is compressed to form a tablet according to known methods, for delivery to the gastrointestinal tract. The tablet composition is typically formulated with additives, (e.g., a saccharide or cellulose carrier) a binder such as starch paste or methyl cellulose, a filler, a disintegrator, or other additives typically usually used in the manufacture of medical preparations. To prepare orally deliverable capsules, protease is mixed with at least one pharmaceutical excipient, and the solid formulation is placed in a capsular container suitable for delivery to the gastrointestinal tract. Compositions comprising a fusion protein are prepared as described generally in Remington, The Science And Practice Of Pharmacy, 21st edition (2005).

As described above, many of the oral formulations of the invention contain inert ingredients, which allow for protection against the stomach environment, and release of the biologically active material in the intestine. Such formulations, or enteric coatings, are well known in the art. For example, tablets containing the TNF-alpha specific protease in admixture with non-toxic pharmaceutically acceptable excipients, which are suitable for manufacture of tablets are used. These excipients are inert diluents, such as calcium carbonate, sodium carbonate, lactose, calcium phosphate or sodium phosphate; granulating and disintegrating agents, for example, maize starch, gelatin or acacia, and lubricating agents, for example, magnesium stearate, stearic acid, or talc.

The tablets aer uncoated or coated with known techniques to delay disintegration and absorption in the gastrointestinal track and thereby provide a sustained action over a longer period of time. For example, a time delay material such as glyceryl monostearate or glyceryl distearate alone or with a wax may be employed.

Formulations for oral use are also presented as hard gelatin capsules, wherein the active ingredient is mixed with an inert solid diluent, for example, calcium carbonate, calcium phosphate, or kaolin or as soft gelatin capsules wherein the active ingredient is mixed with an aqueous or an oil medium, for example, arachis oil, peanut oil, liquid paraffin or olive oil.

Aqueous suspensions contain a TNF-alpha specific protease in the admixture with excipients suitable for the manufacture of aqueous suspensions. Such excipients are suspending agents, for example, sodium carboxymethylcellulose, methylcellulose, hydroxypropylmethylcellulose, sodium alginate, polyvinylpyrrolidone, gum tragacanth and gum acacia; dispersing or wetting agents may be a naturally occurring phosphatide, for example, lecithin, or condensation products of an alkylen oxide with fatty acids, for example, polyoxyethylene stearate, or condensation products of ethylene oxide with long chain aliphatic alcohols, for example, heptadecylethyloxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitol monooleate. The aqueous suspensions also contain one or more preservatives for example, ethyl or n-propel p-hydroxybenzoate, one or more colouring agents, one or more flavouring agents and one or more sweetening agents such as sucrose or saccharin.

Oily suspensions are formulated by suspending the active ingredient in a vegetable oil, for example, arachis oil, olive oil, sesame oil or coconut oil, or in a mineral oil such as liquid paraffin. The oil suspensions may contain a thickening agent, for example, beeswax, hard paraffin or acetyl alcohol. Sweetening agents, such as those set forth above, and flavouring agents may be added to provide a palatable oral preparation. These compositions may be preserved by the addition of an antioxidant such as ascorbic acid.

The pharmaceutical compositions containing the TNF-alpha specific protease are also in the form of oil-in-water emulsions. The oil phase may be a vegetable oil, for example, olive oil or arachis oil, or a mineral oil for example, gum acacia or gum tragacanth, naturally-occurring phosphatides, for example soybean lecithin, and esters or partial esters derived from fatty acids and hexitol anhydrides, for example, sorbitol monooleate, and condensation products of the same partial esters with ethylene oxide, for example, polyoxyethylene sorbitol monooleate. The emulsions may also contain sweetening and flavouring agents.

The proportion of pharmaceutically active TNF-alpha specific protease to carrier and/or other substances varies from about 0.5 to about 100 wt. % (weight percent). For oral use, the pharmaceutical formulation will generally contain from about 5 to about 100% by weight of the active material. For other uses, the formulation will generally have from about 0.5 to about 50 wt. % of the active material.

In other embodiments, oral compositions of the invention are formulated to slowly release the active ingredients, including the fusion proteins of the invention, in the GI system using known delayed release formulations.

In some pharmaceutical formulations of the invention, the fusion protein is engineered to contain a cleavage site between the TNF-alpha specific protease and the second peptide moiety. Such cleavable sites or linkers are known in the art.

Pharmaceutical compositions and methods may include the addition of a transcytosis enhancer to facilitate transfer of the TNF-alpha specific protease across the GI epithelium. Such enhancers are known in the art. See Xia etal., J. Pharmacol. Experiment. Therap., 295: 594-600 (2000); and Xia et al., Pharmaceutical Res., 18(2): 191-195 (2001).

### Detailed Description of the Figures

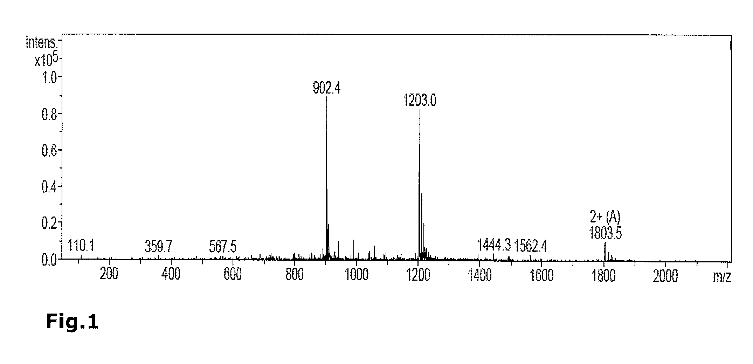

Figure 1: *Mass spectrometric verification of the proteolytic cleavage site of TNF-alpha.* TNF-alpha was digested at a concentration of 1 mg/ml with protease variant E for 1 h at 37 °C in PBS buffer. The short fragment of the cleavage reaction was isolated on a C18-reversed phase HPLC-column and the collected fraction electrosprayed into the orifice of the ion-trap mass spectrometer.

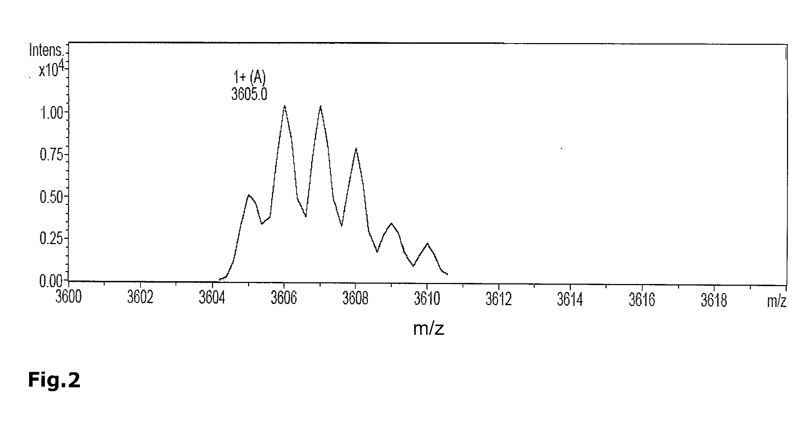

Figure 2: *Deconvoluted mass spectrometric analysis.* The small cleavage product has been isolated and the determined mass represents the first 32 amino acids of SEQ ID NO: 11, confirming the targeted cleavage site.

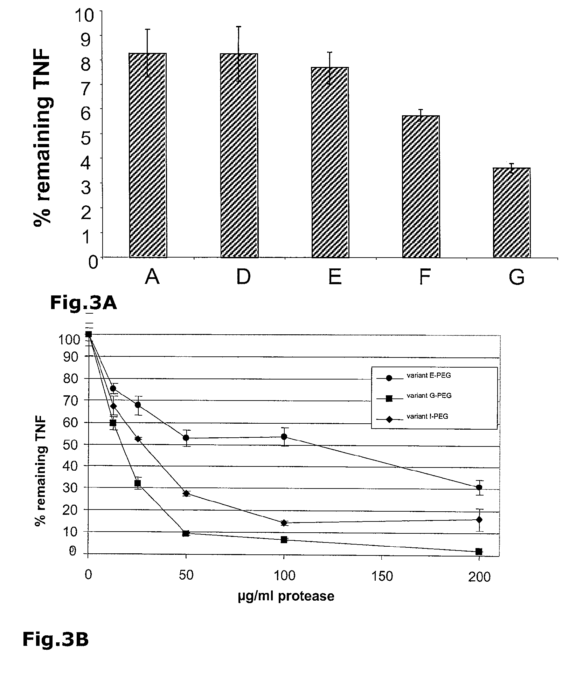

Figure 3: *Inactivation of recombinant human TNF-alpha activity by a panel of variants in a cell-based caspase assay.* The cytokine was pre-incubated at 1 ng/ml for four hours at 37 °C in the presence of the different proteases. Following proteolytic cleavage of hTNFα by the protease, digestions were added to the WEHI 13VAR cells in wells of a microtiter plate. Detection of hTNFα-induced caspase activation was measured by a luminescence based read-out. Data are depicted as remaining TNF-alpha activity as a percentage of activity of undigested TNF-alpha. (A) depicts maximal detected inactivation of hTNFα; (B) depicts dose dependency of the effects of the hTNFα specific protease.

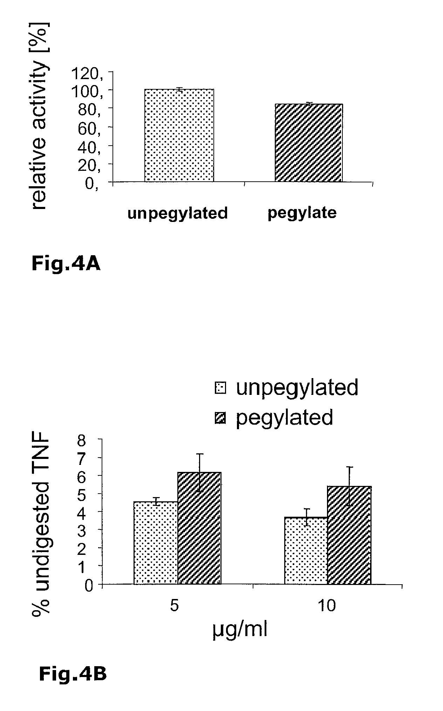

Figure 4: *Effect of pegylation on activity.* Variant E was subjected to protein modification by chemical attachment of 20 kDa poly(ethyleneglycol). The activity of the resulting pegylated protease was assessed against a recombinant hTNFα (A) or by a caspase assay that measures levels of remaining TNF-alpha activity following digestion of the cytokine by the protease (B). Pegylation does not abrogate activity, although a slight reduction in specific activity can be observed.

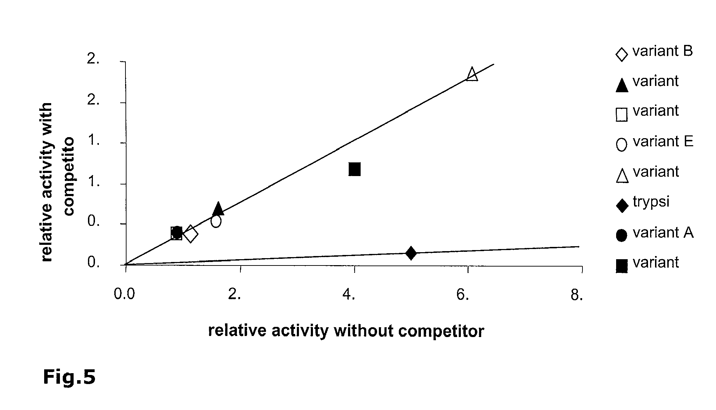

Figure 5: *Specificity measurements of different Variants.* Specificity of the protease variants was compared to unspecific trypsin in a biochemical assay. The substrate is a fluorescently labelled peptide representing the cleavage site in TNF-alpha. Cleavage is assessed by following the changes in the fluorescent signal. To demonstrate the specificity of the variants the assay was performed in the presence and absence of competitor substrate. Specific variants have a higher residual activity in the presence of competitor (protein hydrolysate) compared to activity without competitor. Variant G shows a slightly reduced specificity compared to the other variants, however, wild-type trypsin has a much lower relative activity in the presence of competitor.

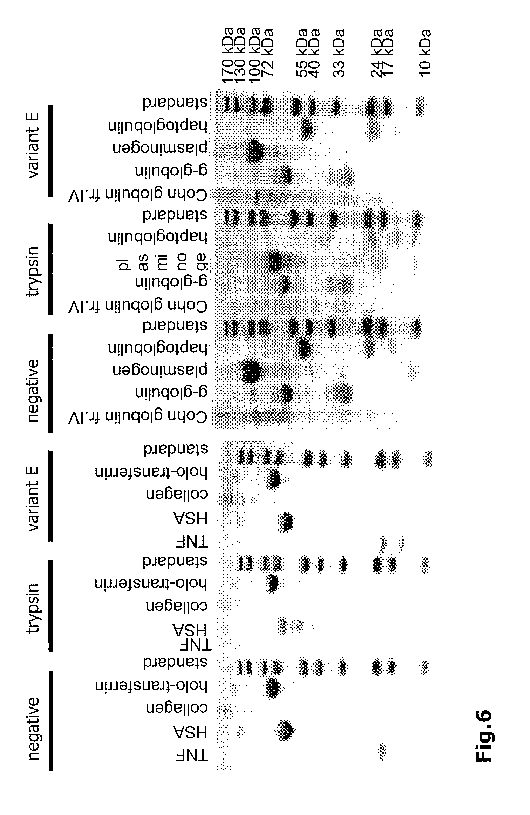

Figure 6: *Proteolytic activity on different serum proteins.* Potential activity towards serum proteins is tested for variant E in comparison with trypsin. The proteins were incubated with protease at a concentration of 50 µg/ml for two hours in PBS buffer, followed by SDS-page analysis of potential cleavage. A close inspection of the gel demonstrates no detectable cleavage of the serum proteins by variant E while substantial degradation is observed in the case of trypsin.

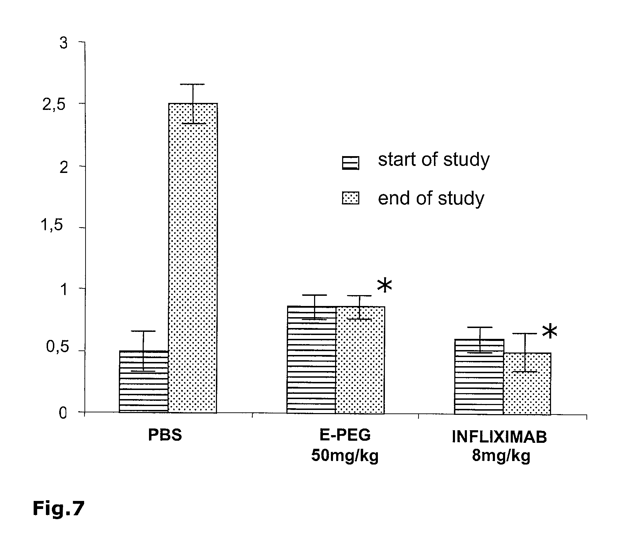

Figure 7: *Efficacy of protease variant in a mouse model.* Efficacy of pegylated TNF-alpha specific protease variant E is demonstrated in a human TNF-alpha transgenic mouse model of arthritis. Six week old transgenic mice (Tg197) were randomly distributed into three dose groups: vehicle alone which served as negative control (PBS), pegylated variant E at 50 mg/kg, and infliximab (Remicade^{®}) at 8 mg/kg which served as positive control. At six weeks of age the mice already expressed mild signs of the disease and hence the study demonstrates efficacy in an established disease setting. Mice were injected three times per week for five weeks. Study was terminated when vehicle control animals showed severe symptoms. Efficacy was determined by using a standard clinical scoring system for ankle joint inflammation. The semi-quantitative scoring system uses a scale of 0 to 3, in which 0 indicates no disease symptoms and 3 extremely debilitating disease symptoms. Asterixes (*) indicate statistically significant differences of specific drug data points when compared to the PBS control (p < 0.01).

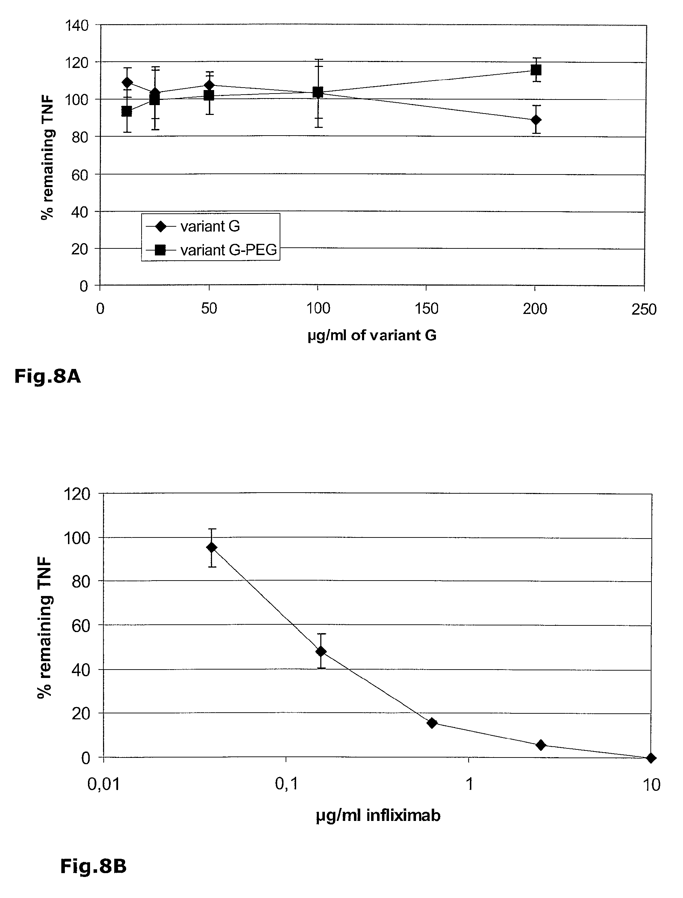

Figure 8: *Membrane-bound TNF-alpha is not cleaved by variant G.* A cell line expressing a variant of human TNF-alpha that is not proteolytically processed and released from the membrane is incubated with an anti-TNF antibody (panel B) or with different amounts of protease variant G or pegylated variant G for 4 h. A reporter cell line WEHI 13VAR is added and apoptosis is induced due to direct contact between membrane-bound TNF and reporter cells.

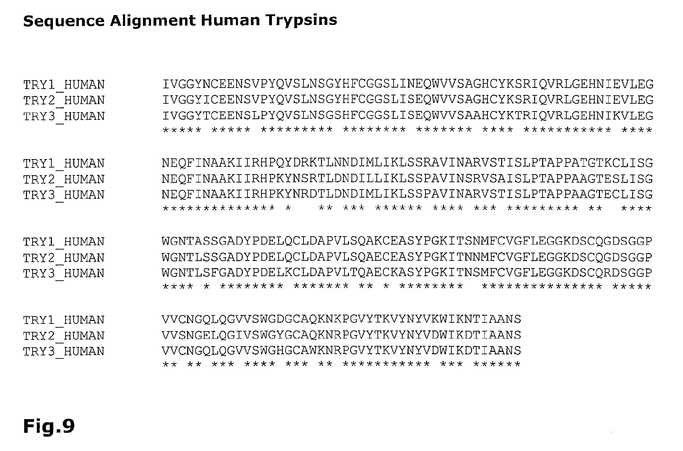

Figure 9: CLUSTAL W (1.7) multiple sequence alignment between human trypsin variants, namely human cationic trypsin (SEQ ID NO:1; top), human Anionic trypsin (Trypsin-2 precursor; SEQ ID NO:25; middle) and human Mesotrypsin (Trypsin-3 precursor, SEQ ID NO:26; bottom). * matching position.

### Examples

### Example 1: Creation of protease variants with alternative specificity determining loops.

Oligonucleotide primer pairs (AF with AR, BF with BR and CF with CR; SEQ ID NOs:27/28, 29/30 and 31/32, respectively) were used to amplify three fragments from the wild-type human trypsin gene. Each pair of oligos (300 nM each) were used with 20 ng trypsin gene template in a PCR using KOD polymerase and the manufacturers recommended buffer. The cycling conditions were 95 °C, 2 min, followed by 25 cycles of 95 °C, 60 s; 58 °C, 45 s and 68 °C, 30 s. Products were purified by standard DNA cleanup columns and the yield and purity assessed by agarose gel eletrophoresis as well as by UV absorbance at 260 and 280 nm. The fragment amplified with BF and BR was used as template (diluted 1:100) in a further PCR containing primers DF and DR (SEQ ID NOs:33 and 34, respectively) in order to introduce the desired variant loops. The PCR and downstream steps were as above. The isolated products from the AF/AR, DF/DR and CF/CR PCRs were then spliced by overlap extension using the above cycling parameters with 40 ng of each fragment and 0.3 µM of the outside primers, AF and CR (SEQ ID NOs:27 and 32, respectively). The product of this reaction was gel isolated and cloned into standard expression vectors, introduced into respective expression hosts and characterized for the protease properties.

In addition, oligonucleotide primers AF and CR (SEQ ID NOs:27 and 32, respectively) were used to amplify 20 ng of wild-type human trypsin as well as proteases containing alternative SDRs using KOD polymerase and the manufacturers recommended buffer. The oligo pair (300 nM each) was used with the cycling conditions 95 °C, 2 min, followed by 25 cycles of 95 °C, 60 s; 58 °C, 45 s and 68 °C, 45 s. Products were purified by standard DNA cleanup columns and the yield and purity assessed by agarose gel electrophoresis as well as by UV absorbance at 260 and 280 nm. The resulting fragment was then amplified with OF and OR using the GeneMorphll kit (Stratagene) as recommended by the manufacturer. The resulting products were gel isolated and cloned into standard expression vectors, introduced into respective expression hosts and characterized for the protease properties.

Individual candidate proteases were isolated according to their enzymatic examined characteristics (e.g., specificity, activity, resistance to inhibitors or inactivation, etc.). Individual genes were cloned into *E. coli* (see Sambrook, J.F., Fritsch, E.F., Maniatis, T., Cold Spring Harbor Laboratory Press, Second Edition, New York (1989)), plated to result in single colonies on solid LB (Luria-Bertani Medium: 1% Trypton, 0.5% yeast extract, 1.0% NaCl with a final pH of 7.0) with 1.5% agar and containing selective marker ("selective solid medium"). An isolated colony was inoculated into liquid LB medium containing selective marker ("selective liquid medium"), and DNA was prepared by Qiaprep Spin DNA Isolation Kit (Qiagen). DNA sequencing reactions were generated using a commercial kit (GenomeLab DTCS Quick Start Kit, Beckman Coulter) and the sequence determined using the CEQ 2000XL DNA Analysis System (Beckman Coulter). The resulting DNA sequence of the gene, through application of the standard genetic code for nuclear genes (see Sambrook, J.F., Fritsch, E.F., Maniatis, T., Cold Spring Harbor Laboratory Press, Second Edition, New York (1989)), unambiguously determined the amino acid sequence of the encoded proteins (SEQ ID NOs:2 through 10).

Selected gene templates were amplified by PCR (see Sambrook, J.F, Fritsch, E.F., Maniatis, T., Cold Spring Harbor Laboratory Press, Second Edition, New York (1989)) using forward and reverse primers (SEQ ID NOs: 14 and 15) resulting after cleavage with restriction enzymes in overhanging ends. After BglI cleavage and ligation (e.g. Sambrook, J.F., Fritsch, E.F., Maniatis, T., Cold Spring Harbor Laboratory Press, Second Edition, New York (1989)) into an *E. coli* cloning vector such as pUC19, the ligated DNA was transformed into E. coli strain Top10F' (Invitrogen) by electroporation. For electroporations into E. coli, commercially available competent cells (Invitrogen) were thawed on ice, mixed with 1 µg DNA and transformed in a 2 mm gap cuvette at 2.5 kV according to the competent cell manufacturer's recommendations. Immediately after electroporation, transformants were supplemented with 1 ml LB liquid medium and incubated for 1 h at 37 °C, followed by plating to solid LB agar containing selective marker ("selective solid medium"). Individual colonies were isolated on selective solid medium to ensure monoclonality. Isolated colonies were inoculated into selective liquid medium, and DNA was prepared by Qiaprep Spin DNA Isolation Kit (Qiagen). DNA sequencing reactions were generated using a commercial kit (GenomeLab DTCS Quick Start Kit, Beckman Coulter) and the sequence was confirmed identical to the above sequence using the CEQ 2000XL DNA Analysis System (Beckman Coulter). After confirmation of the correct DNA sequence, the isolated DNA was transformed into the expression host and protein was prepeared by standard methods as described below. NBE^{®} protease variants were used as culture supernatants or were alternatively purified from the supernatant liquid and used as a purified preparation.

Individual protease variants were purified by a combination of ion-exchange chromatographic steps to 99% homogeniety.

### Example 2: Pegylation

Indidvidual variants were pegylated at a concentration of 1 mg/ml in 150 mM bicarbonate buffer (pH 8.3) with an excess of methoxy-poly(ethyleneglycol)-α-methyl butanoic acid (20 kD, NEKTAR Therapeutics, Huntsville, AL). The activated PEG was added successively over a period of 1 h as solid material. The reaction mixture was stirred for additional 30 min at room temperature. The reaction was stopped by dialysis (3 x 45 min, MWCO 12000) against 20 mM NaOAc, 150 mM NaCl, pH 5.0. The products were separated from non-pegylated protein and unreacted PEG by gel filtration chromatography. The material was dialysed against PBS buffer (MWCO 12000) and lyophilised. This type of chemistry allows covalent coupling of PEG to one or several lysine residues on the surface of the protein.

### Example 3: Mass spectrometric characterization of TNF cleavage product.

In order to verify the targeted cleavage site, TNFα was digested at a concentration of 1 mg/ml with protease variant E (1 mg/ml) for 1 h at 37 °C in PBS buffer. The short fragment of the cleavage reaction was isolated on a C18-reversed phase HPLC-column and the collected fraction electrosprayed into the orifice of the ion-trap mass spectrometer. Spectra were collected and averaged for several minutes (Fig. 1). The deconvoluted mass spectrum shows the isotope pattern and the highest peak roughly represents the average mass of the molecule (Fig. 2). The calculated mass for the fragment is 3606.0 Dalton which is identical with the mass of the third isotope peak. This confirms the cleavage of TNF-alpha after the twin arginine motive by the protease variants. Similar result were obtained with various variants.

### Example 4: Activity of different variants in a TNF-alpha dependent caspase assay.

To assess the hTNFα neutralizing activity in a physiologically relevant manner, a cell-based caspase assay was applied (Caspase-Glo^{®} 3/7 Assay, Promega GmbH, Mannheim, Germany). The assay is carried out following the manufacturer's specifications. The cytokine was pre-incubated at 1 ng/ml for 4 h at 37 °C in the presence of the protease. The concentration of hTNFα used was determined from calibration curves that showed that concentration to result in 90% of maximal sensitivity to cytokine induced caspase activation. Following proteolytic cleavage of hTNFα by the protease, digestions were added to WEHI 13VAR mouse fibroblast cell line using microtiter plates. Optionally, cleavage was performed in human serum. Detection of hTNFα induced caspase activation was measured by a luminescence based read-out (Figs. 3, 4B).

### Example 5: Activity of pegylated material

To demonstrate that the pegylated proteases retain comparable activity against TNF-α in relation to the original, non-pegylated variants, the two forms were compared in parallel in an activity assay against a synthetic peptide (Fig. 4A) or in a caspase assay which measures the functional activity of the cytokine upon binding to its receptor expressed on WEHI 13VAR cells (Fig. 4B). Two concentrations of the protease variants were used in the caspase assay (50 and 100 µg/ml). Overall the results demonstrate that the pegylation has only a marginal effect on the activity of the protease variant.

### Example 6: Demonstrating the specificity of protease variants

Specificity was evaluated by measuring the activity on a target substrate in the presence and absence of competitor substrate. Activity of an unspecific protease is reduced by the competitive substrate, while a specific protease is not or less influenced. Fig. 5 shows the relative specificities of different protease variants. The activity on a peptidic substrate which represents the TNF-alpha cleavage site, is measured in absence and presence of a peptide mixture as competitor substrate. The peptide (20 nM) was incubated with protease in PBS buffer at 37 °C for 10 to 30 min. Cleavage is followed by changes in fluorescence properties of the labelled target peptide. Specificity is expressed as the ratio of cleavage rates in the presence and absence of competitor (slope).

### Example 7: Demonstrating specificity using distinct proteins.

Alternatively to the approach described in example 4 one can compare the activity on distinct proteins. These proteins were chosen according to exposure to the protease variant in the intended therapeutic application and availability. Proteins were digested at a concentration of 1 mg/ml with 50 µg/ml protease in PBS-buffer for 4 h at 37°C and then analysed by SDS-page (Fig. 6). While the unspecific trypsin leads to substantial degradation of the test proteins, for variants E no degradation is detectable.

### Example 8: Efficacy in an animal model.

To validate the efficacy of the TNF-alpha specific protease in vivo, the compound was tested in a human TNF-alpha transgenic mouse model of arthritis. The Tg197 strain has been extensively used to test TNF-alpha biopharmaceuticals (etanercept, infliximab, adalimumab) thus validating the model. Six week old mice were randomly distributed among four groups of 5 to 6 animals each. The mice were treated as follows: phosphate buffered saline (PBS) vehicle alone which served as negative control, pegylated variant E at 50 mg/ml and infliximab at 8 mg/ml which served as positive control. Mice were injected intraperitoneally three times per week for 5 weeks when the animals were sacrificed. Arthritis was evaluated in ankle joints in a blinded manner using a semi quantitative arthritis score ranging from 0 to 3, where 0 means no arthritis (normal appearance and grip strength), 1 indicates mild arthritis (joint swelling), 2 indicates moderate arthritis (severe joint swelling and digit deformation, no grip strength) and 3 indicates severe arthritis (ankylosis detected on flexion and severely impaired movement). At the age treatment started, the mice had expressed clinical symptoms of the disease and as such this example can be used to illustrate the efficacy of the protease in an established disease setting (Fig. 7).

### Example 8: Membrane-bound TNF-alpha is not cleaved.

A cell line expressing a mutant version of TNF with a deletion of amino acids 1 to 12 of the mature soluble protein is incapable of shedding TNF-alpha into solution via cleavage with TNF-alpha converting enzyme (TACE) (Grell et al. (1995). The transmembrane form of tumor necrosis factor is the prime activating ligand of the 80kDa tumor necrosis factor receptor. Cell, 83:793-802). This cell line was either incubated with infliximab or with protease variant G of the invention or pegylated variant G at different concentrations for 4 h. Subsequently a reporter cell line (KYM-1) was added to the wells. The transmembrane TNF induces apoptosis in these TNF-susceptible cells unless it is neutralised by either an antibody or by proteolytic cleavage. Fig. 8B shows the neutralization of TNF by a commercial antibody while variant G or pegylated variant G do not inhibit apoptosis demonstrating that transmembrane TNF-alpha is not inactivated by the protease.

### Sequence Listing, Free Text

- SEQ ID NO: 1: human cationic trypsin
- SEQ ID NOs: 2 to 10: variants E to M
- SEQ ID NO: 11: human TNF-alpha, soluble form
- SEQ ID NO: 12: human TNF-alpha, cloned
- SEQ ID NO: 13: assay peptide, example 4
- SEQ ID NOs: 14 and 15: primers A and B
- SEQ ID NO: 16: general SDR2 sequence
- SEQ ID NO: 17: general SDR3 sequence
- SEQ ID NOs: 18 and 19: SDR3 fragments
- SEQ ID NOs: 20 and 21: SDR2a-b
- SEQ ID NOs: 22 to 24: SDR3a-c
- SEQ ID NO: 25: human Anionic trypsin (Trypsin-2 precursor)
- SEQ ID NO: 26: human Mesotrypsin (Trypsin-3 precursor)
- SEQ ID NOs: 27 to 34: primers AF, AR, BF, BR, CF, CR, DF and DR

## Claims

1. A protease capable of inactivating human tumour necrosis factor-alpha (hTNFα) of SEQ ID NO:10
(a) having a primary structure based on a scaffold derived from wild-type human cationic trypsin having the amino acid sequence shown in SEQ ID NO:1 or from a functional and/or structural equivalent thereof, and
(b) having a first, second and third specificity determining region (SDR) located between residues 21 and 22, 42 and 43, and 123 and 124 in the scaffold, respectively, wherein the numbering refers to the wild-human cationic trypsin as shown in SEQ ID NO:1,
said first SDR having a length of up to 8 amino acid residues and comprising the structure -X₁-X₂-X₃-, wherein X₁, X₂ and X₃ are independently selected from hydrophilic amino acid residues,
said second SDR having the structure -X₄-X₅-X₆-X₇-X₈-X₉- (SEQ ID NO: 16), wherein X₄ is a small or charged amino acid residue, X₅ is P, N or a hydrophobic amino acid residue, X₆ is a hydrophobic amino acid residue, X₇ is P, D, N or a hydrophobic amino acid residue, X₈ is P, T, R or a hydrophobic amino acid residue, and X₉ is A, L or a polar amino acid residue,
said third SDR having the structure -X₁₀-X₁₁-X₁₂-X₁₃-X₁₄- (SEQ ID NO: 17), wherein X₁₀ is L, R or a small amino acid residue, X₁₁ is V, T, S, A, M, G or a positive amino acid residue, X₁₂ is selected from D, A, P, T, N, G, E, L, Q, V, S and R, X₁₃ is selected from F, P, S, D, V, Q, T, R, L, I and Y, and X₁₄ represents a peptidic moiety having one to four arbitrary amino acid residues,
and fragments, regions and derivatives thereof that inactivate hTNFα.

2. The protease of claim 1, which further has a fourth SDR having a length of up to 8, preferably 2 to 5 amino acid residues located between residues 128 and 129 of the scaffold, wherein the numbering refers to the wild-type human cationic trypsin as shown in SEQ ID NO:1, preferably said fourth SDR having at least one P and/or one T residue.

3. The protease of claim 1 or 2, wherein
(i) said first SDR has a length of up to 5 amino acid residues and/or comprises at least two serine residues, preferably X₁ and X₃ are serine residues, most preferably the sequence of said first SDR is SNS or SDS; and/or
(ii) in said second SDR X₄ is a tiny amino acid residue, X₅ is selected from F, A, I, L, P and N, X₆ is selected from F, L, V, W, A, I and G, X₇ is selected from P, M, V, G, D and N, X₈ is selected from A, L, V, F, P, T, R, Y and G, and/or X₉ is selected from D, S, N, T, A, L and E; preferably in said second SDR X₄ is A or S, X₅ is F or L, X₆ is F, L or G, X₇ is P or N, X₈ is A, L, V or G, and/or X₉ is D or E; and/or
(iii) in said third SDR X₁₀ is selected from A, G, S, T, V, R and L, X₁₁ is selected from K, R, S and T, X₁₂ is selected from D, G, L and N, X₁₃ is selected from P, F and I, and/or X₁₄ is an aromatic amino acid residue, L, G, E, S, T or K, or represents a peptide having four amino acid residues, in which peptide the first and second residues are independently selected from charged and small amino acid residues, the third residue is a small or hydrophobic amino acid residue and the fourth residue is a hydrophobic amino acid residue; preferably in said third SDR X₁₀ is A, G, S, or L, X₁₁ is K or R, X₁₂ is D, G or N, X₁₃ is P, and/or X₁₄ is Y, W, RDPY (SEQ ID NO:18) or GALY (SEQ ID NO:19); and/or
(iv) said fourth SDR has the general structure -X₁₅-X₁₆-X₁₇-, wherein X₁₅ is P, X₁₆ is an arbitrary amino acid residue and X₁₇ is T, preferably the sequence of said fourth SDR is PST or PPT.

4. The protease of claim 3, wherein
(i) the sequence of said first SDR is SNS or SDS;
(ii) in said second SDR X₄ is A or S, X₅ is F, X₆ is F or L, X₇ is P, X₈ is A or L, and X₉ is D, preferably said second SDR has the sequence AFFPAD (SEQ ID NO:20) or SFLPLD (SEQ ID NO:21);
(iii) in said third SDR X₁₀ is S, X₁₁ is K, X₁₂ is D, G, or N, X₁₃ is P, and X₁₄ is Y, RDPY (SEQ ID NO:18) or GALY (SEQ ID NO:19), preferably said third SDR has the sequence SKDPY (SEQ ID NO:22), SKGPRDPY (SEQ ID NO:23), or SKNPGALY (SEQ ID NO:24); and/or
(iv) the sequence of said fourth SDR is PST or PPT.

5. The protease according to any one of claims 1 to 4, wherein the protease, in addition to the SDRs, further has one or more amino acid substitutions at the positions 21, 22, 23, 24, 28. 37, 39, 46, 52, 54, 55, 56, 57, 64, 66, 67, 78, 92, 93, 98, 99, 112, 115, 118, 124, 125, 128, 131, 133, 163, 172, 174, 181, 183, 184, 195 and 236, preferably has one ore or more of the following amino acid substitutions (the numbering referring to wild-type human cationic trypsin as shown in SEQ ID:1):
G at position 21 is substituted by A, D, S or V, most preferably by D or V;
Y at position 22 is substituted by T, H, Q, S, W, G or A, most preferably by T or H;
H at position 23 is substituted by T, N, G, D, R or Y, most preferably by T or N;
F at position 24 is substituted by I, V, Q, T, L or A, most preferably by I or V;
S at position 28 is substituted by A;
S at position 37 is substituted by T;
G at position 39 is substituted by S;
I at position 46 is substituted by V, N, L or T, most preferably by V;
E at position 52 is preferably substituted by V or M, most preferably by V;
N at position 54 is substituted by S;
I at position 55 is substituted by T, N or R, most preferably by T or N;
E at position 56 is substituted by G or R, most preferably by G;
V at position 57 is substituted by A, T or G, most preferably by A;
F at position 64 is substituted by I or T, most preferably by I;
N at position 66 is substituted by D;
A at position 67 is substituted by V;
R at position 78 is substituted by W;
S at position 92 is substituted by T;
R at position 93 is substituted by P;
A at position 98 is substituted by D;
R at position 99 is substituted by H;
T at position 112 is substituted by A or P, most preferably by A;
K at position 115 is substituted by M;
I at position 118 is substituted by V;
T at position 124 is substituted by K or I, most preferably byK;
A at position 125 is substituted by P or S, most preferably by P;
G at position 128 is substituted by R, K or T, most preferably by R;
Y at position 131 is substituted by F, N or H, most preferably by F;
D at position 133 is substituted by G;
V at position 163 is substituted by A;
S at position 172 is substituted by T;
Q at position 174 is substituted by R;
V at position 181 is substituted by A;
C at position 183 is substituted by H, Q or R, most preferably by H;
N at position 184 is substituted by K or D;
D at position 195 is substituted by E; and
K at position 236 is substituted by E, D, R, T or V, most preferably by E.

6. The protease according to any one of claims 1 to 5 which comprises a sequence given in SEQ ID NO:2, SEQ ID NO:3, SEQ ID NO:4, SEQ ID NO:5, SEQ ID NO:6, SEQ ID NO:7, SEQ ID NO:8, SEQ ID NO:9 or SEQ ID NO:10.

7. The protease according to any one of claims 1 to 6,
(i) which renders hTNF-α incapable of exerting its biologic effects, preferably is capable of hydrolysing the peptide bonds between positions 31/32 or 32/33 in hTNFα;
(ii) which is a domain of a fusion protein, preferably the fusion protein further comprising domains being selected from binding domains, receptors, antibodies, regulatory domains, pro-sequences, serum albumin, etc.,
(iii) wherein the derivative comprises a covalent linkage to a non-peptide moiety, preferably said non-peptide moiety being selected from polyethylenglycols, carbohydrates, lipids, fatty acids, nucleic acids, metals, metal chelates, nano-particles, liposomes, dendrimers, etc.,
(iv) wherein the functional and structural equivalent of human cationic trypsin is human anionic trypsin und human mesotrypsin shown in SEQ ID NOs:25 and 26, respectively.

8. A nucleotide sequence encoding the protease according to any one of claims 1 to 7.

9. A vector comprising the nucleotide sequence of claim 8.

10. A host cell, cell culture, tissue culture or non-human organism being transformed/transfected with the vector of claim 9 and/or containing the nucleotide sequence of claim 8.

11. A method for the preparation of the protease according to any one of claims 1 to 7, which comprises culturing host cells of claim 10 and isolating the protease from the culture.

12. A pharmaceutical or diagnostic composition comprising the protease according to any one of claims 1 to 7.

13. Use of the protease according to any one of claims 1 to 7 for preparing a medicament for the treatment of clinical conditions resulting from the detrimental activity of tumour necrosis factor.

14. A method for the treatment of clinical conditions resulting from the detrimental activity of tumour necrosis factor in a patient which comprises administering the patient a suitable amount of the protease according to any one of claims 1 to 7.
